(19) **Europäisches Patentamt / European Patent Office / Office européen des brevets**

(11) **EP 4 473 924 B1**

(12) **EUROPEAN PATENT SPECIFICATION**

(45) Date of publication and mention
of the grant of the patent:
**08.04.2026 Bulletin 2026/15**

(21) Application number: **24208754.2**

(22) Date of filing: **13.08.2020**

(51) International Patent Classification (IPC):
*A61B 17/42* *(2006.01)* *A61B 17/32* *(2006.01)*
*A61B 90/30* *(2016.01)* *A61B 17/00* *(2006.01)*
*A61B 17/34* *(2006.01)*

(52) Cooperative Patent Classification (CPC):
**A61B 17/32002; A61B 17/00234; A61B 17/42;**
A61B 17/3468; A61B 90/30; A61B 2017/00026;
A61B 2017/00119; A61B 2017/00287;
A61B 2017/00907; A61B 2017/320024;
A61B 2017/345

(54) **TISSUE REMOVAL SYSTEMS**

GEWEBEENTFERNUNGSSYSTEME

SYSTÈMES D'ÉLIMINATION DE TISSU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.08.2019 US 201962886473 P
07.04.2020 US 202063006360 P**

(43) Date of publication of application:
**11.12.2024 Bulletin 2024/50**

(62) Document number(s) of the earlier application(s) in
accordance with Art. 76 EPC:
**20852209.4 / 4 013 312**

(73) Proprietor: **Claria Medical, Inc.**
**Mountain View, CA 94043 (US)**

(72) Inventors:
• **JONES, Joseph N.**
**Boise (US)**

• **FRANCIS, Daniel E.**
**Mountain View (US)**
• **LE, Kevin**
**Sunnyvale (US)**
• **SALAMINI, Alexey**
**San Francisco (US)**
• **KIM, Steven W.**
**Los Altos (US)**
• **ANDERSON, Scott**
**Sunnyvale (US)**
• **FRENCH, Ronald G.**
**San Jose (US)**

(74) Representative: **SSM Sandmair
Patentanwälte Rechtsanwalt
Partnerschaft mbB
Joseph-Wild-Straße 20
81829 München (DE)**

(56) References cited:
**WO-A1-2016/144170 WO-A1-2018/047180
WO-A1-2019/083896**

## Description

The present application claims the benefit of U.S. Provisional Application No. 62/886,473, filed August 14, 2019, naming Joseph N. Jones et al. as inventors, titled "TISSUE REMOVAL SYSTEMS AND METHODS" and U.S. Provisional Application No. 63/006,360, filed April 7, 2020, naming Joseph N. Jones et al. as inventors, titled "TISSUE REMOVAL SYSTEMS AND METHODS".

## BACKGROUND

In the field of health care in human and veterinary medicine, it is often desirable or even necessary to remove tissue from a patient's body. Such tissue, typically in the form of mass, tumor, or organ, some of which may be benign, cancerous, pre-cancerous, or be suspected of being cancerous or pre-cancerous, may be removed via traditional surgical techniques, including open surgery as well as minimally invasive approaches.

Among minimally invasive approaches, laparoscopic procedures in which a tissue specimen is removed via a small incision using specialized tools are well known. Minimally invasive procedures such as laparoscopy and mini-laparotomy may also employ the use of tools operated robotically. Procedures performed via a minimally invasive approach include those performed in the abdominal, pelvic and thoracic cavities. Cholecystectomies, nephrectomies, colectomies, hysterectomies, myomectomies, oophorectomies, and other procedures in gastrointestinal, gynecological and urological categories are common as are minimally invasive arthroscopy, cystoscopy, and thoracoscopy procedures. Various advantages cited with regard to minimally invasive procedures include enhanced safety, reduced pain, lower risk of infection, shorter recovery times, shorter hospital stays, increased patient satisfaction, and lower cost, among others.

Often, the tissue specimen to be removed via minimally invasive procedures is larger than the incisions used to gain access to the tissue specimen. As such, techniques have been developed to safely remove such specimens while maintaining the advantages of a minimally invasive approach. One such technique is morcellation, in which the tissue specimen is cut or processed into pieces while still inside the patient, or at the level of the skin, or just outside the patient, so that they may be more readily removed. The earliest form of morcellation involved scissors or scalpels to chop up a uterus during vaginal hysterectomy, so that the specimen could be removed through the vagina. Similar manual cutting techniques may be employed when removing many types of tumors or organs through an incision in the abdomen. Later, electromechanical power morcellators were developed that could be deployed via laparoscopic ports which enabled the tissue fragments to be removed via the ports.

In the field of gynecology, the hysterectomy is a common procedure that is performed in approximately 500,000 women per year in the United States alone. The hysterectomy procedure involves removing a woman's uterus, which may be necessary due to any of a variety of reasons, the most common of which (greater than 50% of cases in the U.S.) is due to the presence of uterine fibroids. Uterine fibroids (also known as leiomyoma) are benign tumors which tend to enlarge the specimen, often to the point of where the specimen cannot fit out via the vaginal orifice or minimally invasive surgical incision without the benefit of some form of morcellation. Such hysterectomies may be performed via traditional open surgical techniques or minimally invasive techniques, such as laparoscopy with the use of morcellation or bulk tissue reduction. Hysterectomies may be partial, e.g., involving removal of only the uterus, or total, in which the uterus and uterine cervix are both removed. In either case, the ovaries and/or the fallopian tubes may or may not simultaneously be removed.

For years, power morcellation has been used in gynecologic surgery to remove large uteri from patients via small holes, as is necessary in minimally invasive surgery. The most common application of power morcellation in gynecologic surgery has typically involved morcellating a large, fibroid uterus to remove it from a patient's body during laparoscopic or robotically-assisted laparoscopic hysterectomy, although there are a number of other applications as well - notably myomectomy, in which the uterine fibroids are removed, but the uterus itself is preserved within the patient's body in case the patient desires future fertility.

Since hysterectomy involving an enlarged uterus is very common, and since minimally invasive surgery offers many benefits to the patient, surgeon, hospital, and payer, the use of power morcellation had become commonplace. However, the potential for occult cancers hidden within the uterus that cannot be detected preoperatively and that could potentially be spread around the patient's body with grave consequences during morcellation has been a source of concern. As such, even though most hysterectomies are associated with uteri that do not involve any actual or suspected cancer, traditional open surgery, with its added risk, complication rates, longer hospitalizations, more difficult recoveries, etc., is prevalent. Therefore, techniques and systems are desirable that afford safe removal and processing of tissue specimens, even in the possible presence of an occult malignancy.

WO2018/047180A1, WO2016/144170A1 and WO2019/083896A1 disclose tissue containment and removal systems with contact detection systems.

# EP 4 473 924 B1

SUMMARY

In accordance with the present invention, a tissue containment and removal system includes a tissue container having a conductive layer which comprises a conductive element, an interior volume and an opening. The tissue containment and removal system also includes a bulk tissue reducer having a tissue cutter with a tissue cutting blade that is configured to be conductive. A motor is operatively coupled to the tissue cutting blade of the bulk tissue reducer so as to provide motive force to the tissue cutting blade upon actuation. The tissue containment and removal system further includes a contact detection system having a detection circuit that is operatively coupled to the tissue cutting blade and the conductive element and that is configured to generate a continuity signal between the tissue cutting blade and the conductive element and measure an impedance value between the tissue cutting blade and the conductive element. A controller is operatively coupled to the motor and may be configured to discontinue actuation of the motor and tissue cutting blade whenever the impedance between the tissue cutting blade and the conductive element is at or below a predetermined impedance threshold value.

For some embodiments of a method of containing and accessing a tissue specimen in a patient's body, which does not form part of the present invention, a tissue container may be inserted into the body cavity of the patient. Once the tissue container is disposed within the patient's body cavity, the tissue specimen may be manipulated or otherwise inserted through the opening of the tissue container and into the interior volume of the tissue container. For such manipulation of the tissue specimen, any suitable instruments may be used, such as graspers, tenacula, trocars, cameras and the like, all of which may optionally be inserted into the body cavity through small minimally invasive incisions in the patient's skin and underlying fascia. Once the tissue specimen is disposed within the interior volume of the tissue container, an entire edge of the opening, or rim disposed about the circumference of the opening, of the tissue container may be withdrawn from within the body cavity to a position outside the patient's body which effectively contains the tissue specimen of interest in the interior volume of the tissue container and isolates the tissue specimen from surrounding tissues of the patient's body disposed outside the tissue container. The distal end of a bulk tissue reducer may then be inserted into the interior volume of the tissue container. In some cases, the distal end of the bulk tissue reducer may be inserted into the interior volume of the tissue container until it is adjacent to the tissue specimen. Before, during, or after insertion of the distal end of the bulk tissue reducer into the interior volume, a continuity signal may be transmitted between a tissue cutting blade of the tissue cutter and a conductive element of the tissue container and an impedance between the tissue cutting blade and the conductive element monitored with a detection circuit of a contact detection system. While the impedance between the tissue cutting blade and the conductive element is being monitored by the detection circuit, the tissue cutter of the bulk tissue reducer may be actuated. Thereafter, the tissue cutter may be deactivated by virtue of a deactivation signal from the detection circuit or similar arrangement when the monitored impedance between the tissue cutting blade and the conductive element is at or below a predetermined impedance threshold value.

Some tissue container embodiments which do not form part of the present invention may include an interior volume, an opening and a conductive layer which including a composite weave having conductive strands and non-conductive strands. Some associated embodiments of a method of containing and isolating a tissue specimen within a patient's body may include inserting the tissue container into the body cavity of the patient, the tissue container including an interior volume, an opening and a conductive layer which comprises a composite weave including conductive strands and non-conductive strands. The tissue specimen may then be inserted through the opening of the tissue container and into the interior volume of the tissue container and an entire edge of the opening withdrawn from within the body cavity to a position outside the patient's body.

Some embodiments of a tissue containment and removal system which do not form part of the present invention may include a tissue container having a translucent wall structure and a bulk tissue reducer. The bulk tissue reducer embodiment may include a tissue cutter having a hollow structure with an inner lumen extending a length thereof and a tissue cutting blade disposed at a distal end of the tissue cutter. The bulk tissue reducer may also include a light energy source configured to emit light energy in a distal direction through the inner lumen and from the distal end of the tissue cutter.

Some embodiments of a method of containing and removing a tissue specimen from the patient's body, which do not form part of the present invention, may include inserting the tissue container into the body cavity of the patient, inserting the tissue specimen through the opening of the tissue container and into the interior volume of the tissue container, and withdrawing an entire edge of the opening of the tissue container from within the body cavity to a position outside the patient's body. This method may also include inserting a distal end of the bulk tissue reducer into the interior volume of the tissue container until the tissue cutting blade of the tissue cutter of the bulk tissue reducer contacts the tissue specimen. Light energy is then emitted from a distal end of the tissue cutter in a distal direction towards the tissue specimen in contact with the tissue cutting blade. Light energy leakage may then be observed from between the distal end of the bulk tissue reducer and the tissue specimen. The intensity and orientation of the light energy leakage observed may be used for manipulating the alignment between the distal end of the bulk tissue reducer and the tissue specimen to minimize the amount of light energy leakage between the distal end of the bulk tissue reducer and the tissue specimen.

Some embodiments of a tissue container deployer assembly which do not form part of the present invention may include a tissue container deployer having a sheath with an inner lumen, a rounded distal tip including longitudinal slits that converge together and which form petals in the distal tip of the sheath which are configured to open upon the application of distal axial pressure from within the inner lumen. The tissue container deployer may also include a pusher rod that has an elongate configuration with outside surface which is sized to fit and translate axially within the inner lumen of the sheath and which has an axial length equal to or larger than an axial length of the inner lumen of the sheath. A tissue container embodiment is disposed within the inner lumen of the sheath in contracted state, the tissue container including a wall having a thin flexible configuration, an interior volume, and an opening in communication with the interior volume.

Some embodiments of a method of deploying a tissue container, which do not form part of the present invention, may include inserting a distal end of a sheath of a tissue container deployer assembly through a body opening and into a desired position within an interior cavity of a patient. A pusher rod of the tissue container deployer assembly is axially advanced in a distal direction with respect to the sheath while simultaneously axially advancing the tissue container in the contracted state disposed within the inner lumen of the sheath. The tissue container is so axially advanced with the distal end of the pusher rod which abuts a proximal end of the tissue container. As the pusher rod and tissue container are axially advanced, the method also includes opening flexible petals formed by longitudinal slits in a distal end of the sheath with a distal end of the tissue container to form a distal port in the sheath for distal ejection of the tissue container from the inner lumen of the sheath. The method further includes continuing to axially advance the tissue container with the pusher rod until the tissue container is fully ejected from the distal port of the sheath and into the interior cavity of the patient.

Certain embodiments of the present invention are described further in the following description, examples, claims and drawings. These features of embodiments will become more apparent from the following detailed description when taken in conjunction with the accompanying exemplary drawings.

BRIEF DESCRIPTION OF THE DRAWINGS

FIG. 1 is an elevation view of a tissue containment and removal system embodiment.

FIG. 2 is a top view of a portion of a patient's body with a bulk tissue reducer and tissue container disposed within the patient's body cavity.

FIG. 3 is an elevation view in section of the tissue containment and removal system embodiment of FIG. 1 deployed within the patient's body cavity.

FIG. 4 is a top view of the tissue containment and removal system embodiment of FIG. 1 with the distal end of the bulk tissue reducer disposed within an interior volume of the tissue container.

FIG. 5 is an elevation view of the tissue containment and removal system embodiment of FIG. 4.

FIG. 6 is a perspective view of a drive box embodiment.

FIG. 7 is a top view of the drive box of FIG. 6 with the upper cover removed.

FIG. 8 is a schematic view of a console for the tissue containment and removal system embodiment of FIG. 1.

FIG. 9 is a transverse cross section view of the tissue container of FIG. 1 taken along lines 9-9 of FIG. 1.

FIG. 10 is an enlarged view of the wall portion of the tissue container of FIG. 9 indicated by the encircled portion 10-10 in FIG. 9.

FIG. 11 is an enlarged view of the conductive layer of the tissue container wall of FIG. 10 taken along lines 11-11 of FIG. 10.

FIG. 12 shows an enlarged perspective view of a mesh embodiment.

FIG. 13 is a schematic view of a detection circuit embodiment.

FIG. 14 is an elevation view of a tissue container embodiment that includes a conductive ink disposed on a surface thereof.

FIG. 15 is an enlarged view of the wall portion of the tissue container of FIG. 14 taken along lines 15-15 of FIG. 14.

FIG. 16 is an exploded view of a bulk tissue reducer embodiment.

FIG. 17 is an elevation view of a bulk tissue reducer embodiment in section.

FIG. 18 is a transverse cross section view of the bulk tissue reducer of FIG. 17 taken along lines 18-18 of FIG. 17.

FIGS. 19-21 illustrate a tissue containment method sequence within a body cavity of a patient's pelvic region.

FIG. 22 is an elevation view of a bulk tissue reducer embodiment in use and reducing an isolated tissue specimen from within an interior volume of a tissue container embodiment.

FIG. 23 is an enlarged view of the bulk tissue reducer embodiment of FIG. 17 indicated by the encircled portion 23 and showing a light energy source embodiment and light guide embodiment.

FIG. 24 is an elevation view of a body cavity in a patient's pelvic region with light energy from a light energy source of a bulk tissue reducer embodiment leaking from a gap disposed between the distal end of the bulk tissue reducer and an outside surface of a tissue specimen.

FIG. 25 is an exploded view of a tissue cutter and housing portion of the bulk tissue reducer embodiment of FIG. 1.

FIG. 26 is a perspective view of the tissue cutter of FIG. 25.

FIG. 27 is a perspective view of the tissue cutter of FIG. 26 with a light cone removed in order to expose the light energy source embodiments of the bulk tissue reducer embodiment.

FIG. 28 is a rear view partially cut away of the housing of the bulk tissue reducer embodiment of FIG. 1.

FIG. 29 is an elevation view of a tissue container deployer assembly embodiment.

FIG. 30 is a section view of the tissue container deployer assembly embodiment of FIG. 29 taken along lines 30-30 of FIG. 29.

FIG. 31 is a transverse section view of the tissue container deployer assembly embodiment of FIG. 30 taken along lines 31-31 of FIG. 30.

FIG. 32 is an end view of the sheath embodiment of the tissue container deployer assembly of FIG. 29.

FIGS. 33-35 illustrate a deployment sequence for the tissue container deployer assembly of FIG. 29.

The drawings are intended to illustrate certain exemplary embodiments and are not limiting. For clarity and ease of illustration, the drawings may not be made to scale, and in some instances, various aspects may be shown exaggerated or enlarged to facilitate an understanding of particular embodiments.

DETAILED DESCRIPTION

As discussed above, devices and methods that provide for safe processing and removal of tissue specimens from a position within a patient's body, even in the possible presence of an occult malignancy, may be useful. Certain device and method embodiments for containing and removing tissue specimens from within a patient's body as well as related devices and methods are discussed in U.S. Patent Application No. 16/169,884, titled "Systems and Methods for Tissue Capture and Removal", filed October 24, 2018, by S. Kim et al. and U.S. Patent Application No. 16/758,358, titled "Systems and Methods for Tissue Capture and Removal", filed April 22, 2020, by S. Kim et al. Such devices and methods that function to safely remove tissue specimens from within a patient's body in a minimally invasive manner may be particularly useful. In this specification the non-SI units 'inch' is used, which may be converted to the SI or metric unit according to the following conversion: 1 inch ≈ 25.4 mm.

**EP 4 473 924 B1**

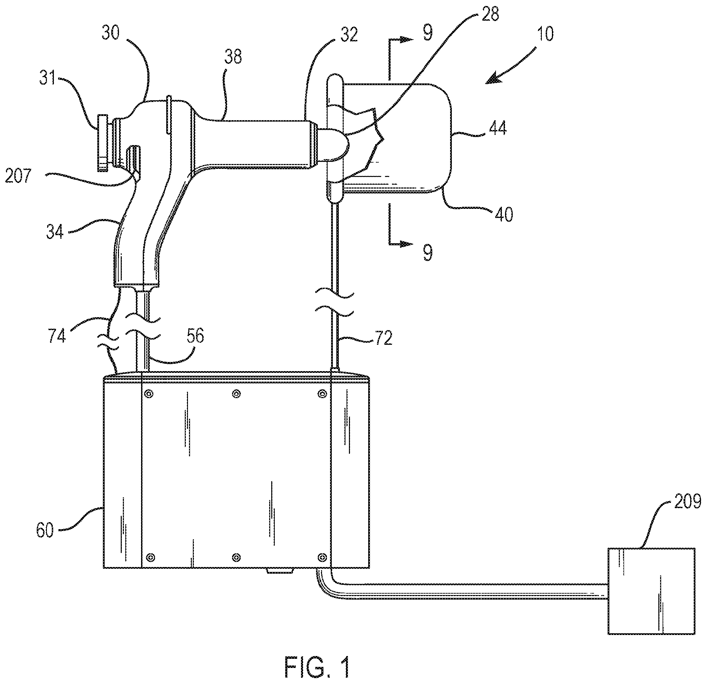

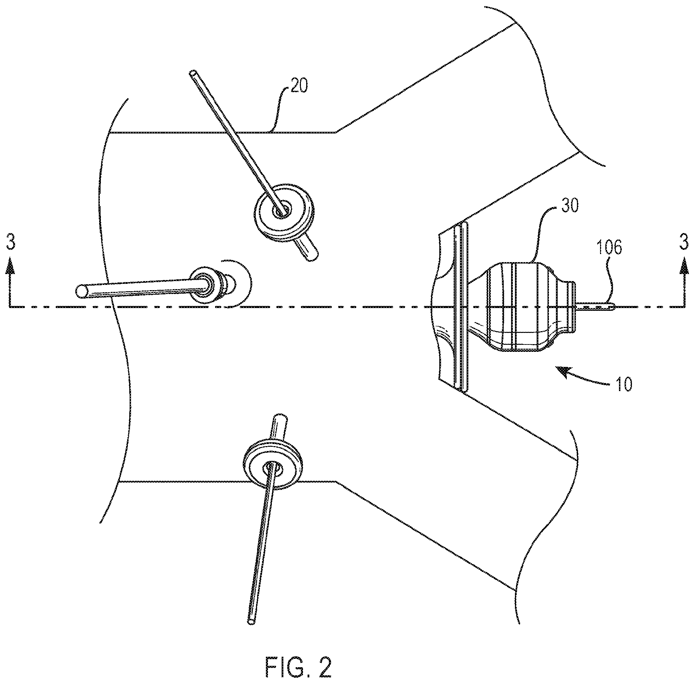

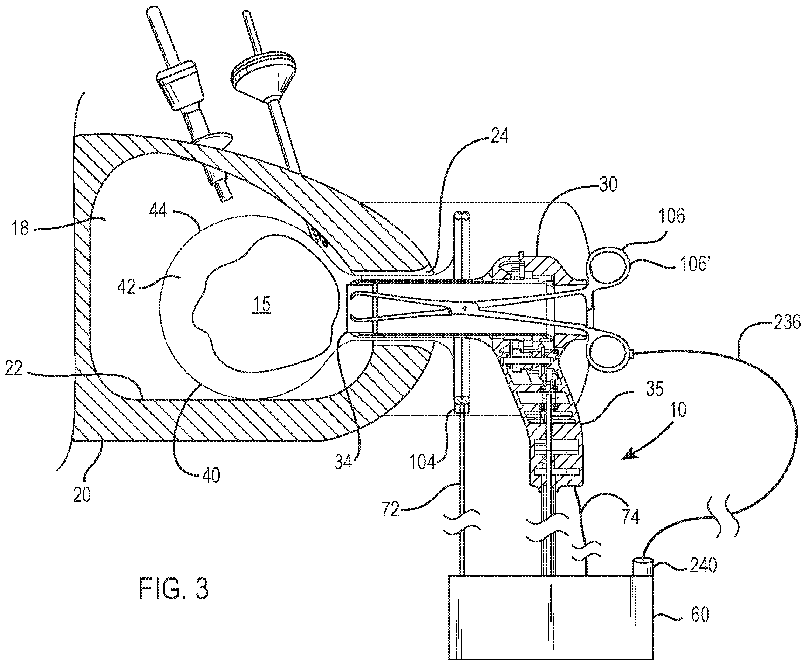

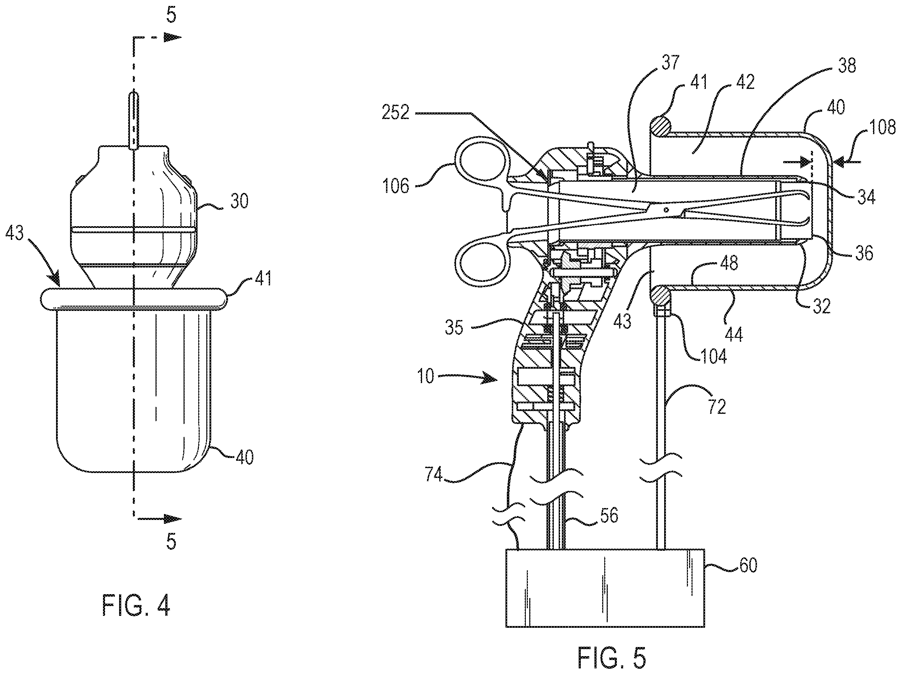

FIGS. 1-3 shows a tissue containment and removal system embodiment 10 that is configured to contain and isolate a tissue specimen 15 in situ within a body cavity 18 of the patient's body 20, and reduce or otherwise morcellate the tissue specimen 15 while the tissue specimen 15 remains disposed within the patient's body 20 and isolated from surrounding tissue 22 within the body cavity 18 as seen in FIGS. 2 and 3. In some cases, the tissue containment and removal system 10 may include devices and associated method embodiments for removing the morcellated tissue specimen 15 from the position within the patient's body 20 accessed through a body opening such as the vagina 24 as shown in FIG. 3. Other body openings 24 suitable for such access may include a surgically created incision in the patient's skin, fascia, internal organs or the like or other natural body openings including the mouth, nostrils or anus. The tissue containment and removal system embodiment 10 shown may include a bulk tissue reducer embodiment 30 (which may also be referred to herein as a tissue morcellator embodiment) and a tissue container embodiment 40. FIGS. 4 and 5 show the bulk tissue reducer 30 of the tissue containment and removal system 10 of FIG. 1 with a distal end 32 of the bulk tissue reducer 30 disposed within an interior volume 42 of the tissue container 40. In order to facilitate introduction of the distal end 32 of the bulk tissue reducer 30 into the interior volume 42 tissue container 40, an obturator 31 may be disposed within an inner lumen or bore 37 of the tissue cutter 34 as shown in FIG. 1. In some cases, the obturator 31 may be generally cylindrical in shape with an outer transverse dimension that is a close sliding fit with the bore 37 of the tissue cutter 34, a length that is at least as great as a length of the tissue cutter 34 and a distal end 28 that has a rounded atraumatic bullet shape that is configured to extend beyond the distal end 33 of the tissue cutter 34 of the bulk tissue reducer 30 and ease introduction of the tissue cutter 34 and associated structures of the bulk tissue reducer 30 into the tissue container 40. Once the distal end 32 of the tissue cutter 34 is appropriately situated within the interior volume 42 of the tissue container 40, the obturator 31 may be proximally withdrawn from the bore 37 and removed from the bulk tissue reducer 30.

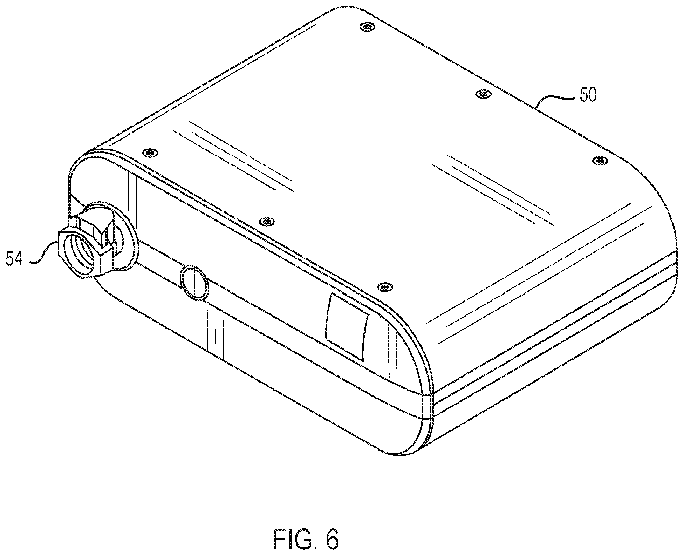

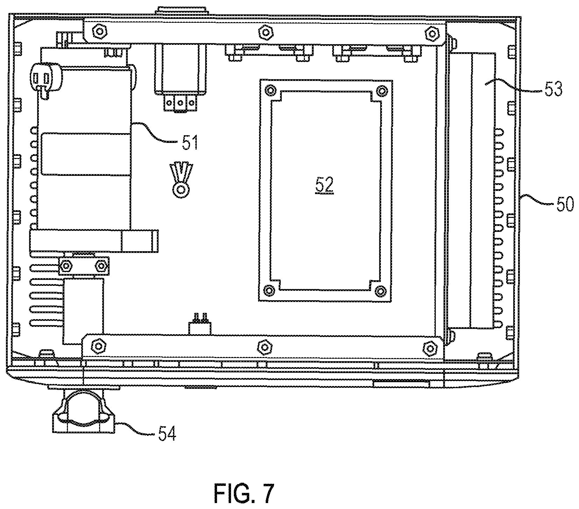

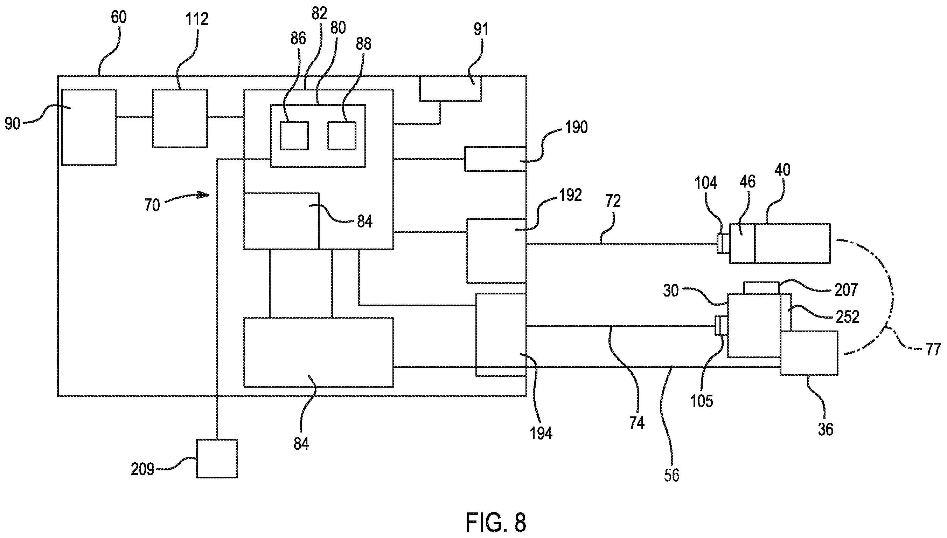

FIG. 6 depicts an embodiment of an optional drive box 50 that may be used for some tissue containment and removal system embodiments 10 to provide rotational energy to a tissue cutter 34 (see FIGS. 3 and 5) of the bulk tissue reducer embodiment 30. FIG. 7 depicts the drive box embodiment 50 without the top cover and illustrates internal component embodiments of the drive box embodiment 50. For some embodiments, the drive box 50 may include a motor 51, a power supply 52, a circuit board 53, and a connector 54 that may be configured for operative coupling to the bulk tissue reducer 30. These components of the optional drive box embodiment 50 may also be included in the console embodiment 60 of the tissue containment and removal system embodiment 10 shown in FIGS. 1 and 8.

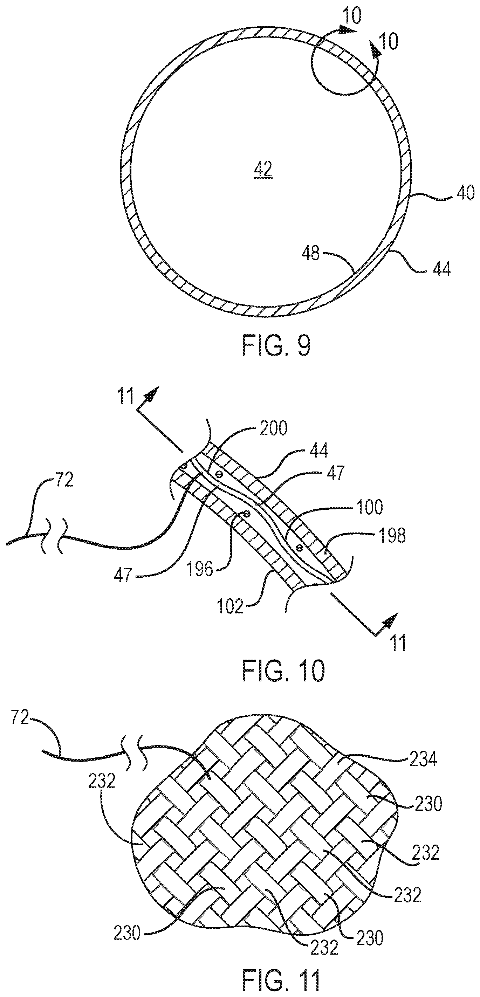

For some tissue containment and removal system embodiments 10, the tissue cutter 34 of the bulk tissue reducer 30 may be operated within the interior volume 42 of the tissue container 40. Because some tissue container embodiments 40 may have a thin flexible wall structure 44, it may be important to prevent contact between the wall 44 of the tissue container 40 and a tissue cutting blade 36 of the tissue cutter 34 that might result in damage to or puncturing of the wall 44 of the tissue container 40. As such, some tissue containment and removal system embodiments 10 may include a contact detection system 70 that may be configured to emit a warning signal and that may optionally include an auto-shut off feature for use when contact or near contact is made with the wall 44 of the tissue container 40 or certain components, such as a conductive element 46 as shown in FIGS. 9 and 10, thereof by the tissue cutting blade 36. More specifically, embodiments of such a contact detection system 70 may be configured to either warn a user of the system when the tissue cutting blade 36 is in contact or close proximity to the wall 44 of the tissue container 40 or shut off power to the motor 51 that is used to rotate or otherwise actuate the tissue cutter 34 altogether in such circumstances.

Although monitoring an electrical impedance 77 between the tissue cutting blade 36 and the conductive element 46 of the tissue container 40 may generally be used to determine proximity or contact between the tissue cutting blade 36 and the wall 44 of the tissue container 40, there are also other modalities and energy types that may be used in contact detection system examples not part of the present invention. For example, time-of-flight optical sensors (not shown) may be used to detect the distance between the tissue morcellator/bulk tissue reducer 30 and the tissue container 40. For such embodiments, a light energy source (not shown) may emit light energy, such as infrared light energy for example, and the infrared optical sensor and an associated controller (not shown) may be configured to measure how long it takes for that infrared light energy to return to the infrared optical sensor. Using the speed of light and the time taken for return of the infrared light energy, such an example may detect how close the bulk tissue reducer is to an inner surface 48 of the interior volume 42 of the tissue container 40. For such a configuration, the longer the time delay, the further away the wall 44 of the tissue container 40 is from the distal end of the tissue cutter 34 of the bulk tissue reducer 30. For some examples, multiple types of sensors may be used with multiple types of light energy emitted and received.

Another exemplary method not part of the present invention of optically detecting proximity or contact between the tissue cutting blade 36 and conductive element 46 of the tissue container 40 may include emitting light energy from a distal end 32 of the bulk tissue reducer 30 or components thereof from a source of light energy (not shown) such as an emitter diode or the like and measuring the amount or amplitude of light energy that is returned to a phototransistor detection sensor. As the tissue cutter 34 of the bulk tissue reducer 30 gets closer to the interior surface 48 of the wall 44 of the tissue container 40, a greater amplitude of light energy or a return light energy signal of greater amplitude will be detected by such a phototransistor type sensor, which may be configured to increase a voltage output of the phototransistor detector. Some such contact detection system examples 70 may be configured to emit a warning or shut off power to the motor 51 of the bulk tissue reducer 30 if a light energy return signal is above a certain predetermined threshold.

Fiber optics (not shown) may also be used to extend the reach of the detector sensor and emitter diode such that the sensors may be placed at an optimum location at the distal end 32 of the tissue cutter 34, or just inside of or just outside of the tissue cutter 34. The inside surface 48 of the tissue container 40 may in some cases be made of a reflective material to enhance the effect of the return light energy signal reflected by the inside surface 48 of the tissue container 40 and to aid in the proximity detection of the bulk tissue reducer 30 or associated cannula 38 (shown in FIG. 5) to the tissue container 40.

A reflective inside surface 48 of the tissue container 40 may also be useful in order to distinguish between the target tissue specimen 15 and the tissue container 40. The amount of light energy returned from the inside surface 48 of the tissue container 40 would be much greater than when detecting tissue, which may allow such a contact detection system 70 to delineate between the two different surfaces or materials thereof. The optical proximity sensor may in turn trigger an example of the contact detection system 70 to determine whether the tissue cutter 34 has come in close proximity or touched a specified layer, such as a conductive layer 47, of the tissue container 40.

In some cases, an ultrasound proximity sensor (not shown) may be used to detect sound waves to detect proximity to the electrically conductive or acoustically sensitive or visible layer of the wall structure 44 of certain tissue container embodiments 40. This in turn may also be used to trigger an example of the contact detection system 70 to determine that the tissue cutter 34 has come in close proximity to the wall structure 44 of the tissue container 40 or touched a specified layer, such as the conductive layer 47, of the tissue container 40.

A capacitive sensor (not shown) may also use electrical capacitance to detect proximity of the tissue cutting blade 36 to the conductive element 46 or conductive layer 47 of the tissue container 40. The capacitive sensor may include a conductive plate and use the sensed object such as the tissue cutting blade 36 or a separate plate structure as the second plate to create the capacitor function. As certain embodiments of the bulk tissue reducer 30 approach such a capacitive sensor, the capacitance value changes which can be used by certain examples of the contact detection system 70 to determine proximity to the sensor and eventually contact with the tissue container 40. Alternately, the capacitive sensor may be built into embodiments of the bulk tissue reducer 30, and potentially use the tissue cutting blade 36 as its plate. When the bulk tissue reducer 30 is brought into proximity with the tissue container 40, the sensor may detect the conductive layer 47 of the tissue container 40 as a second plate of the detection capacitor. The sensors may also be calibrated to distinguish between the metal of the bulk tissue reducer 30 or tissue container 40 and the tissue specimen 15. This in turn may also be used to trigger examples of the contact detection system 70 to determine whether the tissue cutter 34 or tissue cutting blade 36 thereof has come in close proximity or touched a specified layer of the tissue container 40.

Preventing perforation of the wall 44 of the tissue container 40 may also be enhanced by properly aligning the tissue cutting blade 36 of the bulk tissue reducer 30 with the tissue specimen 15 during use. As such, in some cases, certain embodiments of the bulk tissue reducer 30 may include a light energy source such as light-emitting diodes or any other suitable light energy source and a light guide assembly to provide light energy that may travel distally down a bore or inner lumen 37 (see FIG. 5) of the tissue cutter 34 or adjacent structures of the bulk tissue reducer 30 so as to illuminate the tissue specimen 15. In some instances, the light guide may be configured to avoid rotation with the tissue cutting blade 36 of the tissue cutter 34 of the bulk tissue reducer 30, which may be useful for preventing rotation of reduced portions of the tissue specimen 15 disposed inside the inner lumen 37 of the bulk tissue reducer 30 as the tissue specimen 15 (or pieces thereof) is extracted.

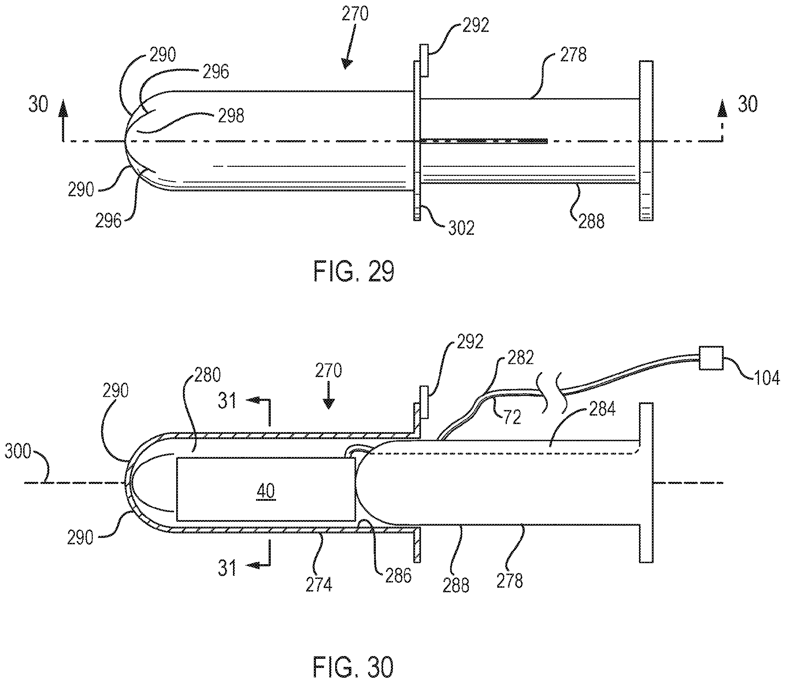

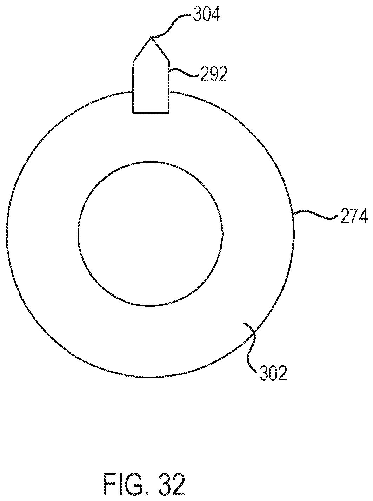

In order to contain and isolate a tissue specimen 15 prior to reduction or morcellation of the tissue specimen 15, it may be desirable to reliably and consistently deploy a suitable tissue container embodiment 40 around the tissue specimen 15 in some cases. This process may typically be carried out in the confined space of the body cavity 18 within the patient's body 20. In some cases, this process may be facilitated by the use of a suitable container deployer or container deployer assembly discussed in more detail below and shown in FIGS. 29-35. In some cases, such container deployer assembly embodiments may be configured to perform deployment of a tissue container 40 while maintaining some control of the orientation of the tissue container 40 during deployment. Some embodiments of such container deployer assemblies may be configured to operate in a manner similar to that of a rivet gun. This type of configuration may be actuated using a spring-loaded mechanism, compressed air, or other mechanical or electrical actuator to deploy the tissue container 40 from a sheath of the tissue container deployer assembly.

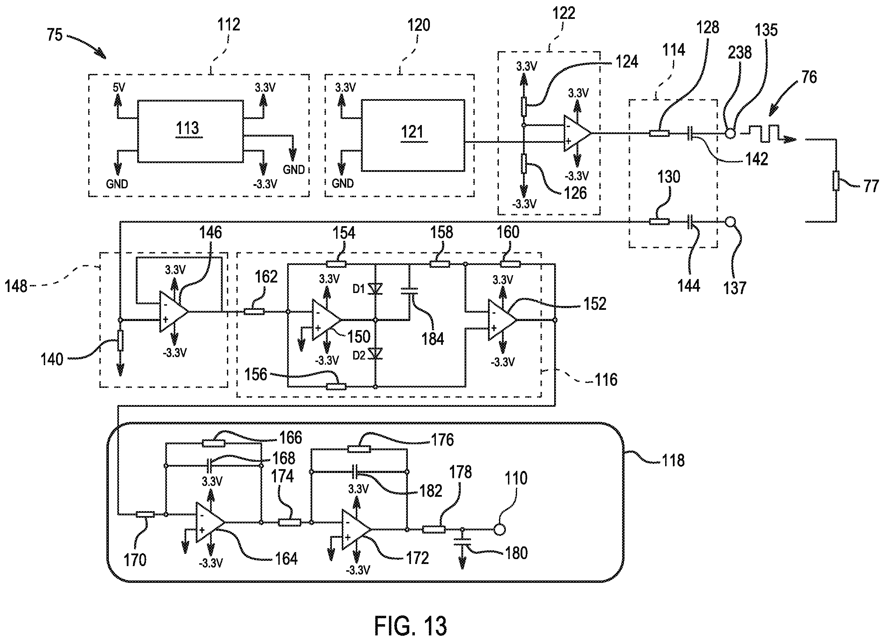

FIG. 3 shows the tissue containment and removal system embodiment 10 of FIG. 1 that includes an embodiment of the contact detection system 70 with an auto-shutoff feature in accordance with the present invention. The tissue containment and removal system 10 is shown with the distal end 32 of the bulk tissue reducer 30 thereof disposed within the interior volume 42 of the tissue container 40 and adjacent a tissue specimen 15 within a pelvic cavity 18 of the patient 20. For such a tissue containment and removal system embodiment 10, by attaching a container conduit 72 (first electrode) in electrical communication with the conductive layer 47 or conductive element 46 the tissue container 40 and a blade conduit 74 (second electrode) to a conductive portion of the tissue cutting blade 36 of the bulk tissue reducer 30, an electrical circuit may be closed when there is physical and electrical contact between the tissue cutting blade 36 and the conductive layer 47 or conductive element 46 of the tissue container 40 and electrical current travels from one to the other. In some cases, this completion of the circuit may be used by a controller 80, as shown in the block diagram of FIG. 8, or a detection circuit 75 thereof as shown in FIGS. 8 and 13, to identify the generally undesirable condition that occurs when the tissue cutting blade 36 of the bulk tissue reducer 30 has come into contact or close proximity with the wall 44 of the tissue container 40.

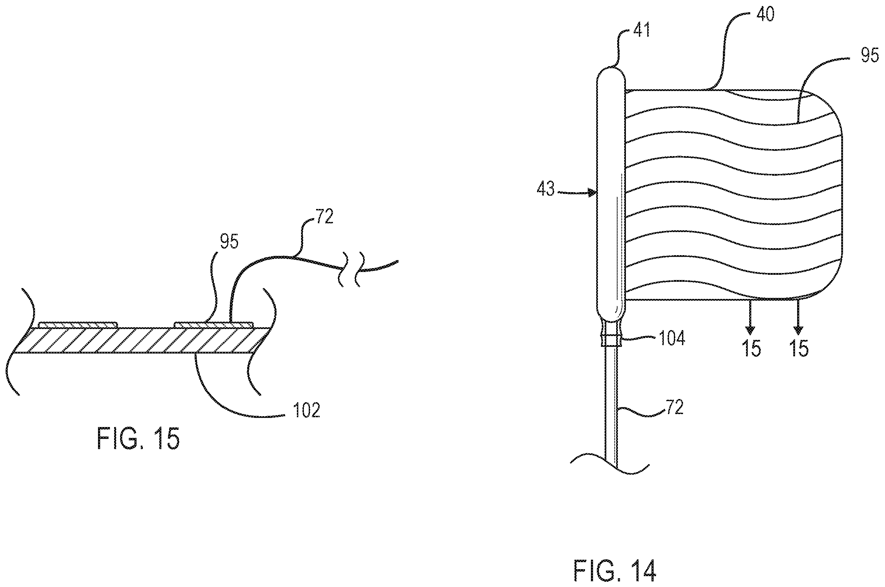

Such contact may, in some circumstances, result in perforation of the wall 44 of the tissue container 40. Perforation of the wall 44 of the tissue container 40 may in some cases defeat the isolation of the tissue specimen 15 or portions thereof disposed within the interior volume 42 of the tissue container 40 from surrounding tissue 22 of the patient 20. The condition of mutual electrical contact, transmission of the continuity signal 76, as shown in FIG. 13, at an amplitude that is at or above a predetermined threshold, or measured effective impedance 77 measured between the conductive element 46 and tissue cutting blade 36 that drops below a predetermined threshold may thus be detected by the controller 80 or contact detection system 70 thereof which may be configured to then emit a warning signal to a user, shut off power to the motor 51 which is coupled to the tissue cutter 34 of the bulk tissue reducer 30 or both of these or to initiate any other useful process upon detection. For some embodiments, the conductive properties of the tissue container 40 may be achieved as a result of the inclusion of a conductive element 46 in the wall structure 44 thereof. Such conductive elements 46 may include a metal mesh layer, a conductive plastic, such as PEDOT or other similar material, conductive plastic mesh or plastic layer in the container construction. In addition, conductive ink 95 including such materials as silver, carbon and the like may be used to create the conducive layer 47 or conductive element 46 of the tissue container 40 as shown in FIG. 14. For some tissue container embodiments 40, the conductive layer 47 may include a woven mesh 100 as seen in FIG. 10 with a composite mesh structure that includes both strands of conductive material and strands of non-conductive material as shown in FIG. 10. In general, conductive layer embodiments 47 and non-conductive layer embodiments (discussed below) of tissue container embodiments 40 discussed herein may include materials such as any suitable biocompatible material, including plastics such as polyethylene, polyurethane, polypropylene, PET, PETG, aramid and para-aramids, including, e.g., poly-paraphenylene terepthalamide (KEVLAR®), aliphatic or semi-aromatic polyamides (NYLON®), Spectra® fibers, rubber, thermoplastics and others.

In some cases, although typically observed as a stepwise function, a drop in the measured impedance 77 and an associated reduction in physical separation between the conductive element 46 of the tissue container 40 and the tissue cutting blade 36 of the bulk tissue reducer 30 may include a predetermined range, enabling the contact detection system 70 to be sensitive enough to stop the tissue cutter 34 of the bulk tissue reducer 30 quickly, but not sensitive enough as to falsely trigger in conductive or aqueous environments. For some embodiments, a non-conductive insulative layer 102 of the tissue container 40 may be disposed between the conductive layer 47 and the bulk tissue reducer 30 as shown in FIG. 10. The continuity signal 76 transmitted between the tissue cutting blade 36 and the conductive element 46 of the tissue container 40 which may be used to determine an impedance value 77 therebetween may include a direct current (DC) or an alternating current (AC) current continuity signal 76 with a frequency ranging from about 1 Hz to about 1 MHz. As shown in FIG. 3, a snap connector 104 may be used that allows the container conduit 72 (first electrode) to be attached into electrical communication with the conductive element 46 of the tissue container 40 after the tissue specimen 15 has been captured and disposed within an interior volume 42 of the tissue container 40. In some cases, the container conduit 72 (first electrode) may be so secured in electrical communication at any time throughout the entire tissue specimen capture process.

For some tissue containment and removal system embodiments 10, portions of various instruments other than the bulk tissue reducer 30 may be electrically insulated so as not to trigger the contact detection system accidentally. For example, in the case of the bulk tissue reducer 30 being used as an antenna, touching the tissue cutting blade with a metal tenaculum instrument 106 might trigger the contact detection system accidentally, whereas by electrically insulating the tenaculum instrument 106 it may be able to interact with the bulk tissue reducer 30 without triggering the contact detection system 70.

As discussed above, the contact detection system 70 of the tissue containment and removal system 10 may include a variety of embodiments. In some cases, the entire bulk tissue reducer 30, or tissue container 40 or conductive layer 47 or conductive element 46 thereof may be configured to function as an antenna that may detect changes to its shape, proximity to other metal elements or other antennae, etc. Additionally, the conductive layer 47 of the tissue container 40, that may include a metal, may be made as a printed flex circuit and metal mesh 100. Using impedance detection properties, the proximity of an object, such as the tissue cutting blade 36, to the conductive element 46 may be detected. This configuration may enable the contact detection system 70 to function such that power can be stopped and the user alerted not just when the tissue cutting blade 36 comes in contact with the conductive layer 47 of the tissue container 40 but also if the tissue cutting blade 36 comes into close proximity with the conductive layer 47 or conductive element 46 thereof. This closeness in proximity or physical separation between the tissue cutting blade 36 of the bulk tissue reducer 30 and the conductive element 46 of the tissue container 40, as may be indicated by associated effective impedance values 77, and that may be used to trigger a detection event may include a proximity value 108 as shown in FIG. 5 of up to about 10 mm, of up to about 1 mm, of about 0.001 mm to about 10 mm, more specifically, about 0.01 mm to about 1mm, and even more specifically, about 0.025 mm to about 0.075 mm.

Referring to FIGS. 8 and 13, the contact detection system embodiment 70 is shown that may be built into a console printed circuit board (PCB) 82 and coupled to the conductive element 46 of the tissue container 40 and the tissue cutting blade 36 of the bulk tissue reducer 30. In some embodiments, detection may be performed by outputting a square wave embodiment of the continuity signal 76 with a frequency of at least about 20kHz and a magnitude of about +3.3V to -3.3V in electrical communication with the tissue cutting blade 36 of the bulk tissue reducer 30. Some such continuity signal embodiments 76 may also have a voltage of up to about 5 V in some cases. When the tissue cutting blade 36 makes contact with the conductive element 46 in the wall 44 of the tissue container 40, the resulting reduction in effective impedance 77 therebetween and corresponding increase in continuity signal transmission is received back at the console PCB 82 through the conductive conduit 72 such as a wire connected between the console PCB 82 and the conductive element 46 of the tissue container 40.

In some configurations, the continuity signal 76 may be outputted to the tissue container's conductive element 46 and then received through a handpiece 35 of the bulk tissue reducer 30. A sine wave, triangular wave or other waveform may be used instead of a square wave in some cases. In some instances, a sine wave may create less electrical noise, but also less signal to detect. In some instances, the contact detection system 70 may be configured to generate a continuity signal 76 with high frequency alternating current, which in some cases may be about 20kHz, because lower frequencies may be more likely to induce cardiac arrhythmias. By increasing the frequency to about 20kHz or more in some cases, the contact detection system 70 may safely transmit an embodiment of the continuity signal 76 with up to about 10mA of current through the body 20 of a human patient. In some cases, for reference, the safe current flow limit according to an electrical safety standard - IEC 60601-1 may be up to about 50uA when DC current is used. For some embodiments, the design of the detection circuit 75 shown in FIG. 8 may be configured to limit current amperage of the continuity signal 76 to a maximum of up to about 6.6mA at a frequency of at least about 20kHz or more.

For the detection circuit embodiment 75 shown in FIG. 13, the impedance 77 between the conductive element 46 of the tissue container 40 and tissue cutting blade 36 as measured by the detection circuit 75 is effectively processed and converted by the detection circuit 75 to a Vout signal at a Vout terminal 110 that generates a voltage output having an amplitude that is inversely related to the measured impedance 77. That is, the lower the measured impedance 77, the higher the Vout signal at the Vout terminal 110 generated by the detection circuit 75. As such, it has been empirically found that in certain embodiments of the contact detection system 70, when the tissue cutting blade 36 of the bulk tissue reducer 30 was disposed in and surrounded by air in the middle of the interior volume 42 of a tissue container 40, a Vout signal of < 50 mV was measured at the Vout terminal 110 by a detection circuit 75 of the contact detection system 70 which is essentially the Vout signal associated with an open circuit or nearly infinite impedance 77 measured between the conductive element 46 of the tissue container and tissue cutting blade 36.

When the tissue cutting blade 36 of the bulk tissue reducer 30 was put in direct contact with a stainless steel conductive element 46 in the wall 44 of the tissue container 40, a Vout measurement at the Vout terminal 110 of about 2.1 V to about 2.2 V was made, which is indicative of a Vout signal consistent with a very low or near zero impedance measured between the conductive element 46 of the tissue container 40 and the tissue cutting blade 36. When the tissue cutting blade 36 of the bulk tissue reducer 30 was placed in contact with the non-conductive inner layer 102 of the container wall 44, a measurement of about 500 mV was made. This is generally interpreted to indicate that there is some capacitive coupling between the conductive element 46 of the container wall 44 and the tissue cutting blade 36 of the bulk tissue reducer 30, even when no direct electrical contact is made with the conductive element 46 in the all of the tissue container 40.

In another test, the non-conductive inner wall 44 of the tissue container 40 was scratched to expose some of the conductive stainless steel mesh 100 of the conductive element 46. Tap water and saline were used to fill the interior volume 42 of the tissue container 40 and the tissue cutting blade 36 of the bulk tissue reducer 30 was dipped into the water. A Vout reading at the Vout terminal 110 of about 1.2 V to about 1.6 V was made on the detection circuit embodiment 75. This reading reflects current flow of the continuity signal 76 from the tissue cutting blade 36 of the bulk tissue reducer 30 passing through the saline and into the conductive stainless steel mesh 100 of the conductive element 46 of the wall 44 of the container 40. The different readings seem to generally indicate that the contact detection system embodiment 70 tested may be used to differentiate between electrical contact between the tissue cutting blade 36 of the bulk tissue reducer 30 and conductive element 46 of the container wall 44 (about 2.1V) and contact between the tissue cutting blade 36 of the bulk tissue reducer 30 and tissue/saline/body fluids in the tissue container 40 with exposed conductive elements 46 (from previous contact with the blade for example) at about 1.4 V.

For some embodiments, the contact detection system 70 may have 4 main subsystems including a power supply 112, a patient interface 114, a receiver/rectifier 116 and a signal filtering unit 118. Referring to FIG. 13, for some embodiments, the power supply 112 may include a power supply circuit including a positive and negative output charge pump and evaluation module 113 such as Texas Instruments model LM27762EVM which may be configured to convert a 5V input voltage into a +3.3 V output and a -3.3V output which may be used to supply electrical power to the rest of the detection circuit 75 of the contact detection system 70. The detection circuit 75 may also include a signal generator 120 that may include an analog device 121 such as an analog device model DC20738-H manufactured by Analog Devices Corporation located at Norwood, MA, may be configured to generate a square wave signal at a frequency of about 20 kHz and serve as a signal generator for the detection circuit 75. In some cases, a continuity signal at a frequency of about 10 kHz to about 30 kHz may be generated by such an analog device. The square wave output may be used to feed into a comparator 122 which is powered by the +3.3V and -3.3V rails of the charge pump 113 so that the output swings between +3.3V and -3.3V at 20kHz. Resistor R1 124 and resistor R2 126 may be selected to give a threshold for the switching at nominally one half of the +3.3 V supply. For the exemplary embodiment shown, the resistors 124 and 126 may have a resistance of about 10 kohms each.

The patient interface portion 114 of the detection circuit may include resistor R3 128 and resistor R4 130 which may be selected to limit the patient auxiliary current per IEC 60601-1 Table 3, requirements for type body floating (BF). For some embodiments, resistors 128 and 130 may have a resistance of about 499 ohms each. In some cases, a 100 μA limit for low frequency AC current may be increased based on the elevated 20kHz or greater transmitter/signal generator frequency. The limit for low frequency AC current may be increased over two orders of magnitude to become about a 10mA limit in some cases. In some cases, the maximum voltage between the transmit terminal or blade terminal 135, which may be coupled to the blade conduit 74, and the receive terminal or container terminal 137, which may be coupled to the container conduit 72, may be set to about 6.6V (i.e., the difference between the 3.3 V negative output and 3.3 V positive output). Leakage current values may include the 6.6V divided by the series resistors including resistor R3 128 and resistor R4 130: $i_{leakage}$ = 6.6 V / (499 + 499) = 6.6mA. This configuration may be used to achieve compliance within safe physiological boundaries without factoring in resistor R5 140 which further limits the current. For some exemplary embodiments, resistor R5 140 may have a resistance of about 249 ohms. Capacitor C1 142 and capacitor C2 144 may be used to block DC current from flowing to the patient's body. The capacitance values of capacitor C1 142 and capacitor C2 144 may also be selected to minimize droop when the square wave is either high or low. For the exemplary embodiment shown, the capacitors 142 and 144 may have a capacitance of about 10 μF each.

The receiver/rectifier circuit portion 116 of the detection circuit 75 of the contact detection system 70 may include an amplifier such as an operational amplifier U1B 146 which may be configured to act as a buffer/follower 148 simply passing the voltage from the top of resistor R5 140 to the next stage. Resistor R3 128, resistor R4 130, resistor R5 140 and the measured resistance 77 (Rmeas) may be configured to form a resistor divider. An amplifier such as operational amplifier U2A 150 and operational amplifier U2B 152 may be configured to form a rectifier, which flips the negative portion of the continuity signal to become positive such that it looks more like a DC output with the exception of the square wave edges. This rectifier may have the ability to add gain to the output of the resistor divider discussed above. If, for example, the resistance for resistor R7 154, resistor R8 156, resistor R9 158, and resistor R10 160 are the same value, then the equation for the Rmeas 77 to Vout 110 may be as follows:

$$Vout = 0.5*6.6*(R5/(R3+Rmeas+R4+R5))*(R7/R6)$$

For some embodiments, the resistance value of the resistors 154, 156, 158 and 160 may be equal to each other and be about 2.2 kohms. In some instances, resistor values may be selected based on use during certain conditions, such as when the non-conductive inner lining 102 of the tissue container 40 has been nicked and there is tissue between the bulk tissue remover 30 and the conductive element 46 exposed by the nick. In such circumstances, the resistance between the bulk tissue remover 30 and the conductive layer 47 of the tissue container 40 may be approximated by about 1 kohm. It may be useful in some such circumstances to maximize the difference in Vout 110 for an Rmeas 77 of 0 ohms and 1 kohm subject to two design considerations including the rail voltage. More specifically, high gain (large resistance values for resistor R7 154 and resistor R6 162) may allow a large voltage difference between Vout 110 for 0 ohms and 1 kohm but the rails prevent unlimited use of gain. For some embodiments, the resistance value for resistor 162 may be about 687 ohms. Operational amplifier performance at high gain may also need to be considered under such parameters. Another design consideration includes noise immunity. Using a resistor R5 140 of a relatively small resistance may allow for larger gain given fixed voltage rails, but it may also make the voltage divider input voltage small relative to potential noise sources. Certain values for certain detection circuit embodiments 75 may be selected as follows: Vout 110 at 0 ohms = 2.1V, Vout at 1 kohms = 1.2V and Vout 110 at high-Z < 50 mV.

Embodiments of a filter circuit of the signal filtering circuit 118 may include an amplifier such as an operational amplifier U3A 164 which may be configured as an inverting active low pass filter with a role-off frequency determined by resistor R12 166 and capacitor C4 168 and a gain configured by resistor R11 170 and resistor R12 166. For such filter circuit embodiments, a filtering value of F3db = 1 / (2 * pi * R12 * C4) may be achieved, with a gain of -R12/R11. An amplifier such as an operational amplifier U3B 172 may also be configured as an inverting active low pass filter. A filtering value of F3db = 1 / (2 * pi * R14 * C5), which is also a function of capacitor C5 182, which may have a capacitance of about 10 nF, may be achieved, with a gain of - R14/R13, each a function of resistor R13 174 and/or resistor R14 176. Finally, resistor R15 178 and capacitor C6 180 may be configured to form a low pass filter with a role off frequency: F3db = 1 / (2 * pi * R15 * C6). For some embodiments, capacitor 180 may have a capacitance of about 10 nF. If all three filters are configured with the same resistor-capacitor (RC) values, the filter may be configured to aggressively roll off. In such a circuit embodiment the role-off frequency may be about 15.9 kHz. Another design consideration of setting an aggressively low cut off frequency is delay. In some cases, the delay may be approximated by about 3 time constants of 63 microseconds, for a total delay of about 189 microseconds for this particular exemplary embodiment. For the embodiment shown, the resistors 166, 170, 174, 176 and 178 may all have the same resistance value of about 1 kohm. The exemplary embodiment of the detection circuit 75 discussed above also includes a pair of diodes D1 and D2 as shown, as well as capacitor C3 184 which may have a capacitance of about 100 pF. In addition, the operational amplifier embodiments 146, 150, 152, 164, and 172 as well as the amplifier of the comparator portion 122 may all include operational amplifier model OPA2192, manufactured by Texas Instruments, in Dallas Texas.

Some embodiments of the tissue containment and removal system 10 may include the tissue container 40 having the conductive layer 47 which includes the conductive element 46, an interior volume 42 and an opening 43. The tissue containment and removal system 10 may also include the bulk tissue reducer 30 having the tissue cutter 34 with the tissue cutting blade 36 being configured to be conductive. A motor 51 may be operatively coupled to the tissue cutting blade 36 of the bulk tissue reducer 30 so as to provide motive force to the tissue cutting blade 36 upon actuation, which, in some instances, may be a rotational motive force. The tissue containment and removal system 10 may further include the contact detection system 70 having a detection circuit 75 that is operatively coupled to the tissue cutting blade 36 and the conductive element 46 and that is configured to generate a continuity signal 76 between the tissue cutting blade 36 and the conductive element 46 and measure the impedance value 77 between the tissue cutting blade 36 and the conductive element 46. The controller 80 may be operatively coupled to the motor 51, and may be configured to discontinue actuation of the motor 51 and tissue cutting blade 36 which is operatively coupled thereto whenever the impedance 77 between the tissue cutting blade 36 and the conductive element 46 is at or below a predetermined impedance threshold value. Once the controller 80 discontinues actuation of the motor 51 because the impedance 77 between the tissue cutting blade 36 and the conductive element 46 is at or below a predetermined impedance threshold value, the controller 80 may set a latch of the discontinued power state that will remain in place, regardless of changes to the measured impedance value 77 subsequent to the shutdown, until a reset command is issued by the user of the system 10.

In some instances, the controller 80 may be optionally be configured to actuate or otherwise emit a warning signal whenever the impedance 77 between the tissue cutting blade 36 and the conductive element 46 is at or below the predetermined impedance threshold value through an indicator 190. For some embodiments, it may be desirable to emit an audible warning signal from an audible signal emitter, in which case the indicator 190 may include a speaker. In such cases, the controller 80 may be configured to actuate the audible warning signal from the audible signal emitter whenever the impedance 77 between the tissue cutting blade 36 and the conductive element 46 is at or below the predetermined impedance threshold value. For some embodiments, it may be desirable to emit a visual warning signal from a visual signal emitter. In such cases, the indicator 190 of the contact detection system 70 may include a light energy source such as an LED light or any other suitable source. In such cases, the controller 80 may be configured to actuate a visual warning signal from the visual signal emitter of the indicator 190 whenever the impedance 77 between the tissue cutting blade 36 and the conductive element 46 is at or below the predetermined impedance threshold value.

Referring to FIGS. 8 and 13, in some cases, the contact detection system 70 may include the blade terminal 135 that is operatively coupled to the controller 80 and to the tissue cutting blade 36 with a conductive blade conduit 74, and the container terminal 137 may be operatively coupled to the controller 80 and to the conductive element 46 of the tissue container 40 with the conductive container conduit 72. For some of these embodiments, the conductive blade conduit 74 that operatively couples the blade terminal 135 and tissue cutting blade 36 may have a snap connector 105 that is configured to provide a releasable electrical coupling therebetween. For some of these embodiments, the conductive container conduit 72 that operatively couples the container terminal 137 and conductive element 46 of the tissue container 40 may also have the snap connector 104 that is configured to provide a releasable electrical coupling therebetween. In addition to the snap connectors 104, 105, the contact detection system may also include a container connection interface 192 disposed in communication between the container conduit 72 and the controller 80 and a bulk tissue reducer connection interface 194 disposed in communication between the blade conduit 74 and the controller 80.

The controller may include the console printed circuit board 82 and a motor driver 84 operatively coupled to the motor 51. The controller 80 may further include the signal generator 120, a processor 86 and a memory 88 operatively coupled to the processor 86. The contact detection system 70 may also include a main power supply 90 operatively coupled to the controller 80 and/or any other suitable components of the contact detection system 70 as well as a cooling fan 91 that may be useful in order to keep the components within the console 60 at a proper operating temperature. The motor 51 may be operatively coupled to the tissue cutting blade 36 of the bulk tissue reducer 30 by a flexible shaft 56 which is configured to transmit rotational torque from the motor (or a suitable gear system coupled thereto) to the tissue cutter 34 of the bulk tissue reducer 30.

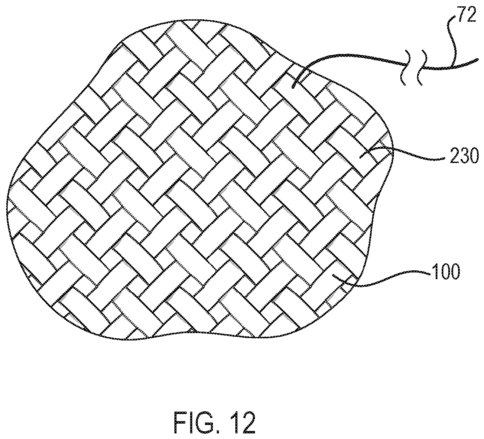

Referring to FIGS. 9 and 10, for some tissue containment and removal system embodiments 10, the tissue container 40 may include a second non-conductive layer 102 which is disposed on an inside surface 196 of the conductive layer 47. The tissue container 40 may also additionally include a third non-conductive layer 198 disposed on an outside surface 200 of the conductive layer 47. The conductive layer 47 disposed between the second layer 102 and the third layer 198 may include a composite weave having non-conductive strands interwoven with conductive strands which make up the conductive element 46 of the tissue container 40. For some embodiments, the conductive layer 47 disposed between the second layer 102 and third layer 198 may also include a thin flexible layer of non-conductive polymer material with a pattern of conductive ink 95 as shown in FIG. 14. The conductive ink 95 may be flexible after printing which may produce a wall structure 44 with a flexible configuration. For such embodiments, the conductive ink 95, which may be printed onto an inside surface of the wall 44, an outside surface of the wall 44 or in any other suitable location. For some embodiments, a print pattern of the conductive ink 95 may include a pitch that ensures that the tissue cutting blade 36 of the bulk tissue reducer 30 will contact the conductive ink 95 prior to perforating the thin polymer layer that the conductive ink 95 is printed on. For such tissue container embodiments, the conductive ink 95 may serve as the conductive element 46 of the tissue container 40. For some other embodiments, the conductive layer 47 and conductive element 46 thereof disposed between the second layer 102 and the third layer 198 may include a wire mesh 100 made entirely from conductive strands 230 as shown in FIG. 12. The conductive strands 230 of the wire mesh 100 may include or be made from metals like stainless steel in some instances.

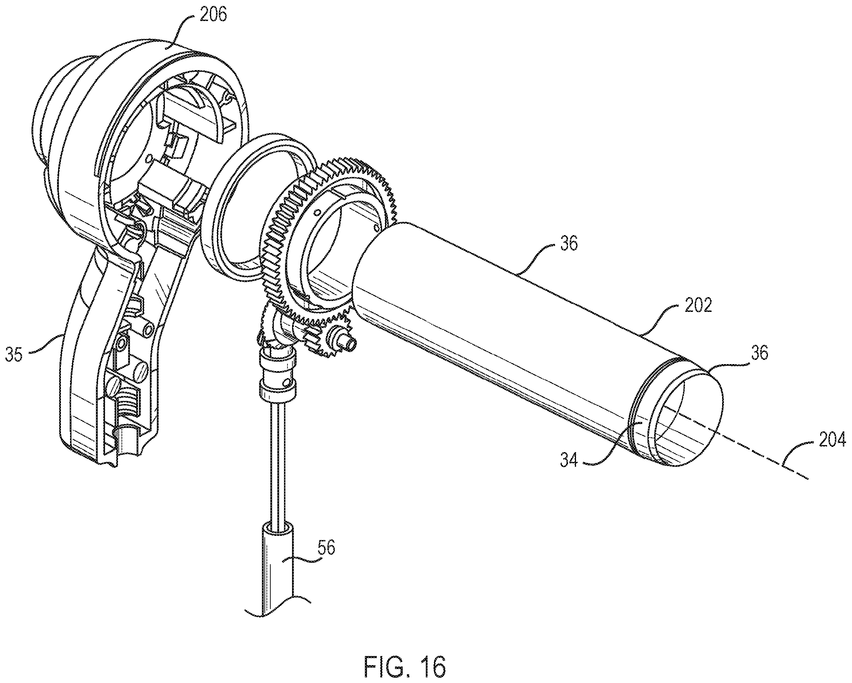

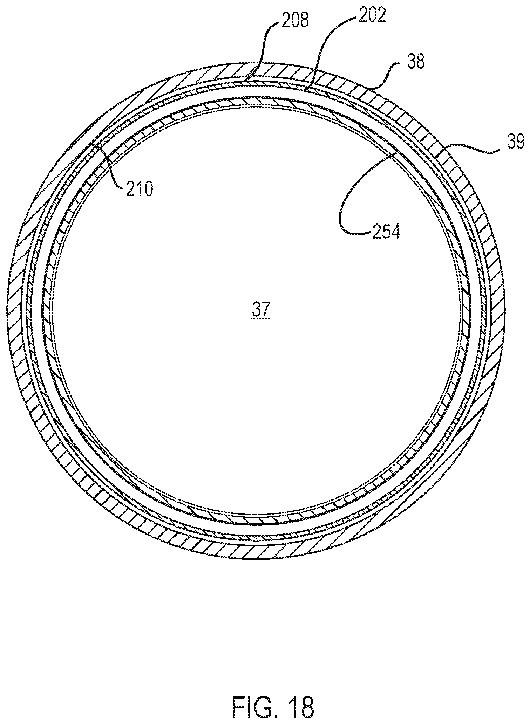

Referring to FIGS. 16-18, the tissue cutter embodiment 34 shown includes an elongate tube 202 having an inner lumen 37 extending a length thereof. For some embodiments, the tissue cutting blade 36 includes a sharpened distal end of the elongate tube 202 of the tissue cutter 34 that has a beveled configuration, although alternative configurations may be used. For the embodiment shown, the shape of the tissue cutting blade 36 is circular in a transverse section profile with the entire tissue cutting blade 36 lying in a plane which is perpendicular to a longitudinal axis 204 of the elongate tube 202 of the tissue cutter 34. The elongate tube 202 of the tissue cutter 34 may be made from any suitable high strength material that may also optionally be conductive. Metals such as nickel titanium alloy, stainless steel and the like may be used in some cases. For the illustrated embodiment, the bulk tissue reducer 30 includes a housing 206 which is operatively coupled to the tissue cutter 34 and a cannula 38 which is secured to the housing 206. Such a cannula embodiment 38 may have an elongate hollow configuration and include an inner lumen 39 that extends a length thereof. As shown, the cannula 38 is disposed over the elongate tube 202 of the tissue cutter 34 with a close fit between the outside surface 208 of the elongate tube 38 and the inside surface 210 of the cannula 38. For some bulk tissue reducer embodiments, a manual activation switch 207 may be disposed on the housing 206 and be operatively coupled to the controller 80 in order to manually actuate the motor 51 and tissue cutter 34 of the bulk tissue reducer 30. The system 10 may also include a footswitch 209 which is operatively coupled to the controller 80 in order to actuate the motor 51 and tissue cutter 34 of the bulk tissue reducer 30 as shown in FIG. 8.

As discussed above with regard to the particulars of exemplary detection circuit embodiments 75, the proximity value 108 of the tissue cutting blade 36 with respect to the conductive element 46 of the tissue container 40 may be represented by various corresponding measured parameters such as impedance 77, measured current flow of the continuity signal 76, Vout signal from the Vout terminal 110 etc. For some embodiments, the impedance threshold value may be selected to correspond to a proximity value 108, which indicates the distance of separation between the tissue cutting blade 36 and the conductive element 46, of up to about 1 mm. In order to provide a clinically safe continuity signal, in some cases, the detection circuit 75 may be configured to generate a continuity signal 76 including an alternating current having a frequency of about 10 kHz to about 30 kHz, more specifically, about 20 kHz, a maximum current flow of up to about 10 mA, a square wave continuity signal or any suitable combination of these parameters.

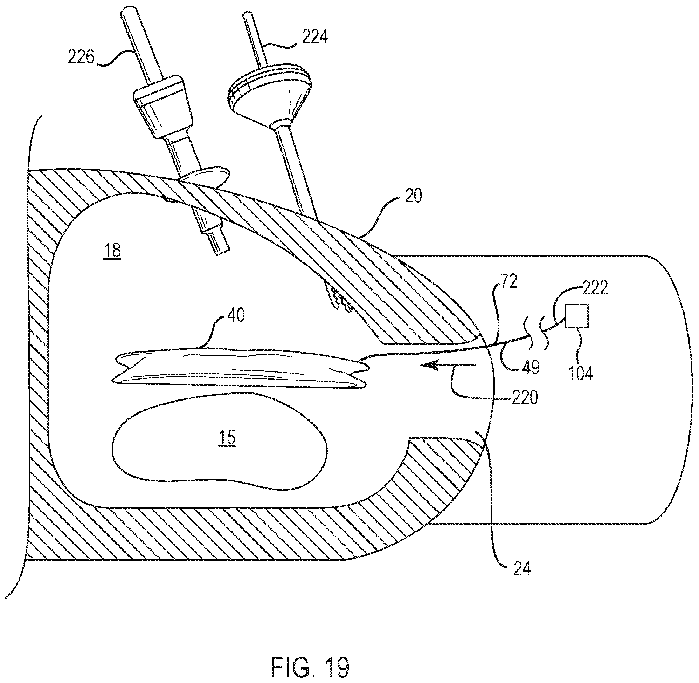

For some tissue specimen removal procedures, the tissue container 40 may be inserted into the body cavity 18 of the patient 20 as shown in FIG. 19 and as indicated by arrow 220. For some embodiments, a tether 49 having a distal end thereof secured to the rim 41 of the tissue container may have a proximal end 222 thereof remaining outside of the patient's body cavity 18. For some embodiments, the tether 49 may also include a conductive conduit so as to serve as the container conduit 72. For such embodiments, a snap connector 104 may be operatively coupled to the proximal end 222 of the tether 49.

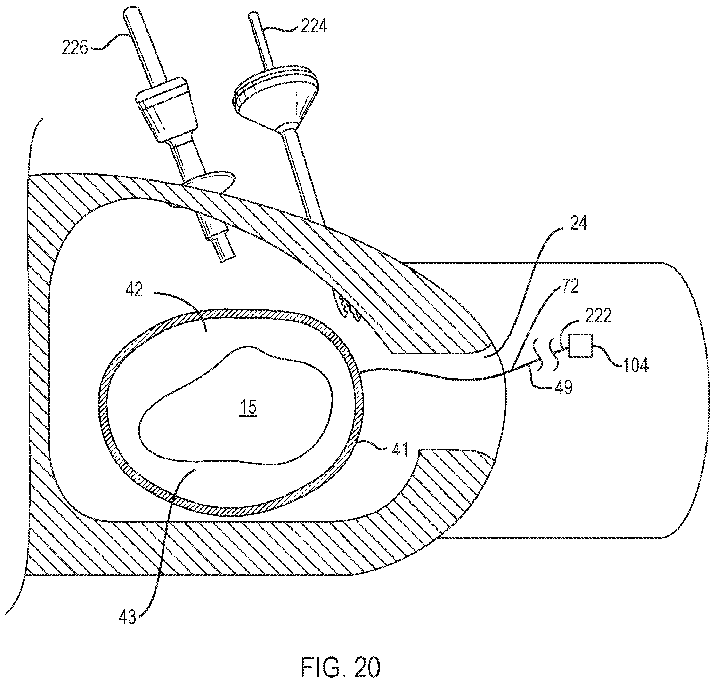

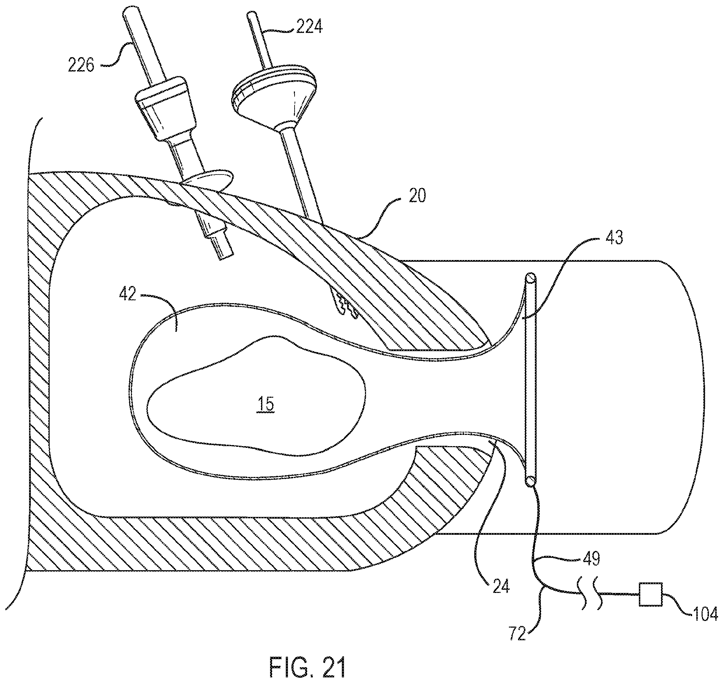

Once the tissue container 40 is disposed within the patient's body cavity 18, the tissue specimen 15 may be manipulated or otherwise inserted through the opening 43 of the tissue container 40 and into the interior volume 42 of the tissue container 40 as shown in FIG. 20. For such manipulation of the tissue specimen, any suitable instruments may be used, such as graspers 224, cameras 226, tenacula 106, trocars (not shown), and the like, all of which may optionally be inserted into the body cavity 18 through small minimally invasive incisions in the patient's skin and underlying fascia. Once the tissue specimen 15 is disposed within the interior volume 42 of the tissue container 40, an entire edge of the opening 43 as defined by the rim 41 disposed about the circumference of the opening 43 may be proximally withdrawn from within the body cavity 18 to a position outside the patient's body 20 while the tissue specimen 15 simultaneously remains within the interior volume 42 and within the patient's body cavity 18. This arrangement of the tissue specimen 15, tissue container 40 and body opening 24 effectively contains the tissue specimen 15 of interest in the interior volume 42 of the tissue container 40 and isolates the tissue specimen 15 from surrounding tissues 22 of the patient's body 20 disposed outside the tissue container 40 as shown in FIG. 21. At this point, the snap connector 104 of the tether 49 may be operatively coupled to the container terminal 137.

The distal end 32 of a bulk tissue reducer 30 may then be inserted into the interior volume 42 of the tissue container 40 and into the interior cavity 18 of the patient's body 20. In some cases, the distal end 32 of the bulk tissue reducer 30 may inserted into the interior volume 42 of the tissue container 40 until it is adjacent the tissue specimen 15 as shown in FIG. 3. During, before, or after insertion of the distal end 32 of the bulk tissue reducer 30 into the interior volume 42, the continuity signal 76 may be transmitted between a tissue cutting blade 36 of the tissue cutter 34 and the conductive element 46 of the tissue container 40 and an impedance 77 between the tissue cutting blade 36 and the conductive element 46 monitored with the detection circuit 75 of a contact detection system 70. While the impedance 77 between the tissue cutting blade 36 and the conductive element 46 is being monitored by the detection circuit 75, the tissue cutter 34 of the bulk tissue reducer 30 may be actuated. Thereafter, the tissue cutter 34 may be deactivated by virtue of a deactivation signal from the detection circuit 75 or similar arrangement when the monitored impedance 77 between the tissue cutting blade 36 and the conductive element 46 is at or below a predetermined impedance threshold value.

For some embodiments, in addition to deactivating the tissue cutter 34 when the monitored impedance 77 between the tissue cutting blade 36 and the conductive element 46 is at or below a predetermined impedance threshold value, an audible warning signal may also be emitted by the detection circuit 75, controller 80 or any other suitable component when the monitored impedance 77 between the tissue cutting blade 36 and the conductive element 46 is at or below a predetermined impedance threshold value. In some cases, emitting the audible warning signal may include emitting a beep tone. For some embodiments, the beep tone may be configured to conform to IEC 60601-1-8 specifications with regard to maximum and minimum volume, frequencies or any other applicable parameters. Also, in addition to deactivating the tissue cutter 34 when the monitored impedance 77 between the tissue cutting blade 36 and the conductive element 46 is at or below a predetermined impedance threshold value, a visual warning signal may also be emitted by the system 70 when the monitored impedance 77 between the tissue cutting blade 36 and the conductive element 46 is at or below a predetermined impedance threshold value. For some embodiments, the visual warning signal emitted may include a light signal. In addition, for some continuity detection methods, the impedance threshold value may be determined to correspond to a proximity value 108 indicating a physical distance between the tissue cutting blade 36 and the conductive element 46 as shown in FIG. 5. For some embodiments, this corresponding proximity value 108 may be up to about 1 mm. As such, with this type of configuration, the tissue cutter 34 of the bulk tissue reducer 30 may be deactivated when the proximity value 108 between the tissue cutting blade 36 and the conductive element 46 is up to about 1 mm. In some cases, other proximity values 108 corresponding to respective impedance threshold values may include a proximity value 108 of up to about 10 mm, up to about 1 mm, about 0.001 mm to about 10 mm, more specifically, about 0.01 mm to about 1mm, and even more specifically, about 0.025 mm to about 0.075 mm.

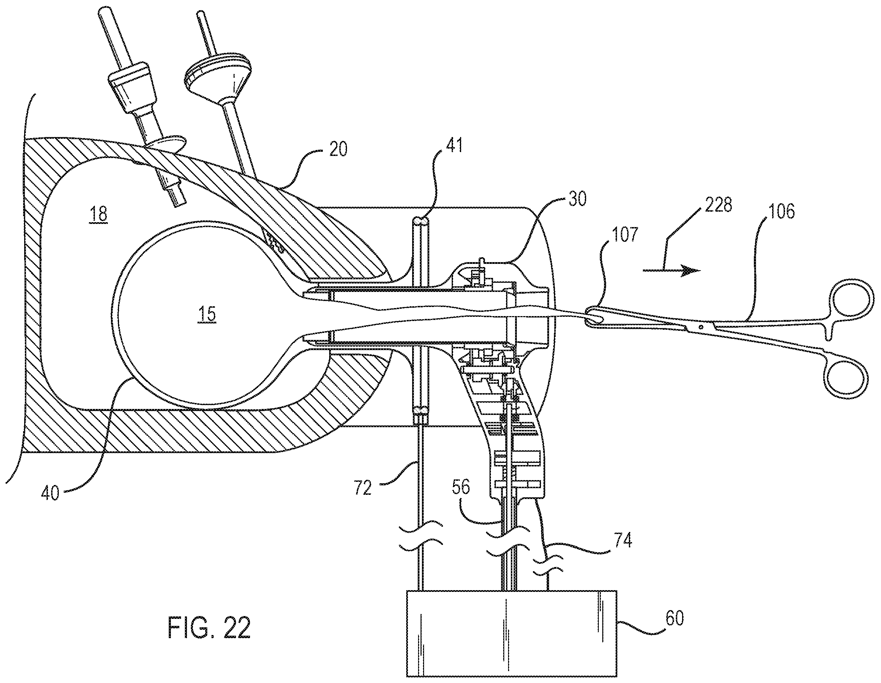

In some cases, prior to deactivation of the tissue cutter 34, the tissue specimen 15 may be contacted with the tissue cutting blade 36 of the tissue cutter 34 and the tissue specimen 15 reduced with the actuated tissue cutter 34 as shown in FIG. 22. During such reduction of the tissue specimen 15, a distal end 107 of the tenaculum 106 may be secured to the tissue specimen 15 and the tissue specimen 15, or grasped reduced portions thereof, pulled proximally (as indicated by arrow 228) through the inner lumen 37 of the tissue cutter 34 while reducing the tissue specimen 15 with the actuated tissue cutter 34. The reduced tissue specimen 15 may continue to be withdrawn through the inner lumen 37 of the tissue cutter 34 until at least a portion of the tissue specimen 15 is disposed outside of the bulk tissue reducer 30 and the patient's body 20. The process may be continued, in some cases, until the entire tissue specimen 15 has been removed from the interior volume 42 of the tissue container 40.

With regard to grasping the tissue specimen 15 with the distal tips of the tenaculum 106, in some instances the tissue to be grasped 15 may be disposed inside of the bore 37 of the tissue cutter 34 or distally adjacent the distal end 32 of the tissue cutter 34. More specifically, referring to the tissue containment and removal system embodiments 10 of FIGS. 3 and 5, a tenaculum embodiment 106 is shown that has an axial length that is substantially similar to an axial length of the bulk tissue reducer 30. For such embodiments, the distal tips of the tenaculum 106 may be substantially coextensive with the distal end 32 of the tissue cutter 34 when the distal tips of the tenaculum 106 have been fully advanced into the bore 37 and are at their maximum distal extension within the bore 37 as shown. For such system embodiments 10, in order to grasp a tissue specimen 15 with the distal tips of the tenaculum 106, the tissue specimen 15 may be pressed against the distal end 32 of the tissue cutter 34 such that at least a portion of the tissue specimen 15 extends proximally into the bore 37 forming a "tissue meniscus" (not shown) that may be grasped by the distal tips of the tenaculum 106 while still in the bore 37. In other cases, the tenaculum 106 may have an axial length that allows the distal tips of the tenaculum 106 to extend distally from the distal end 32 of the tissue cutter 34. For some embodiments, the tenaculum 106 may have an axial length that allows the distal tips of the tenaculum 106 to extend distally from the distal end 32 of the tissue cutter 34 by a distance of up to about 25 mm or more. For such embodiments, a tissue specimen 15 disposed outside of the bore 37 but distally adjacent the distal end 32 of the tissue cutter 34 may still be grasped by the distal tips of the tenaculum 106.

In some cases, transmitting the continuity signal 76 between the tissue cutting blade 36 of the tissue cutter 34 and the conductive element 46 of the tissue container 40 may include transmitting a continuity signal 76 including an alternating current having a frequency of about 10 kHz to about 30 kHz, more specifically, about 20 kHz, a maximum current flow of up to about 10 mA, a square wave continuity signal or any combination of these or other suitable parameters.

For some tissue container embodiments, the stainless steel mesh 100 may be used as a reinforcing layer of the tissue container 40 and, that reinforcing layer may serve as the conductive element 46 in the wall 44 of the tissue container 40 used for completing an electrical detection circuit 75 for the contact detection system 70 as shown in FIGS. 9-11. If a generally non-conductive reinforcement layer 102 which may also be used as a fluid tight or sealing layer (a polyester weave, kevlar® weave, spectra® weave, or just a tough polymer layer) is used for the tissue container 40 then an additional conductive element 46 and/or conductive layer 47 may be included in the wall 44 of the tissue container 40 in order to be used for completing the detection circuit 75 for detection by the contact detection system 70. The conductive element 46 may include conductive strands 230 in some cases which may be woven into a weave with non-conducting strands 232 to form a composite weave 234 that may serve as the conducting layer 47 of the container 40. For such composite weave embodiments, the conductive strands 230 may form an interwoven overlapping grid type pattern wherein adjacent and overlapping conductive strands 230 make electrical contact with each other such that all portions of all conductive strands have electrical continuity with each other. In this way, a continuity signal 76 being transmitted to any portion of any conductive strand 230 will be communicated to all portions of all other conductive strands 230 of the composite weave 234. For these embodiments 234, the container conduit 72 may be electrically coupled to any portion of any of the conductive strands 230 and achieve electrical continuity with all portions of all other conductive strands 230 of the composite weave 234.

The conductive element 46 may also include a stretchable conductive material or materials which may screen printed or stamped or pad printed onto a surface of a polymer layer or other form of otherwise non-conductive layer of the tissue container 40 in some cases. The conductive element(s) may also be manufactured in a similar manner to a flexible circuit board. As an example, the conductive ink 95 as shown in FIG. 14 may be screen printed onto an outside surface of an inner or first layer of some container embodiments or any other suitable layer. In some container embodiments 40, the material of the conductive ink 95 may be placed in contact with a kevlar® weave or another polymer film layer disposed on an outside surface of the conductive ink material 95. Such a multiple layer configuration may be laminated together in some cases.

In some cases, the conductive middle layer 47 of a multi-layered tissue container wall 44 may be made of a composite weave 234 of strands of different materials as discussed above. Such a composite weave 234 may in some cases be made of non-conductive polyester strands, polyethylene strands, and conductive metal strands including stainless steel strands, or any combination of metal strands and polymer strands. The ratio of non-conductive polymer strands 232 to conductive metal strands 230 in such composite weave embodiments 234 may range from about 20% to about 80% or from about 10% to about 90% or from about 1% to about 99%. For some exemplary embodiments, the ratio of conductive metal strands 230 to non-conductive polymer strands 232 may be between about 1% metal and about 99% polymer, about 10% metal and about 90% polymer, about 20% metal and about 80% polymer, about 30% metal and about 70% polymer, about 40% metal and about 60% polymer, about 50% metal and about 50% polymer, about 60% metal and about 40% polymer, about 70% metal and about 30% polymer, about 80% metal and about 20% polymer, about 90% metal and about 10% polymer, about 99% metal and about 1% polymer or any other ratio in between these ranges. For some composite weave embodiments 234, a ratio of the number of conductive strands to the number of non-conductive strands may be about 5% to about 20%, more specifically, about 8% to about 12%.

Using synthetic polymer strands 232 in place of steel/metal strands 230 may improve flexibility of the woven composite layer 234 compared to a mesh layer made completely of metal strands 230 and potentially cut resistance without adding weight. Conductive fibers 232 may also be woven into largely non-conductive materials to facilitate the function of contact detection system embodiments 70 including auto shutoff features in the tissue containment and removal system embodiments 10 discussed herein.

FIG. 14 depicts stretchable conductive ink 95 printed onto a portion of the tissue container 40. The conductive ink 95 may be used as the conductive element 46 of the tissue container 40 to detect contact with the bulk tissue reducer 30 and trigger the detection circuit 75. The conductive ink 95 may be printed in a location disposed between two layers 102, 198 of non-conductive polymer material such as plastic so that the conductive ink 95 is not exposed on the inside surface or outside surface of the tissue container 40.

FIG. 10 shows how the middle layer 47 of a layered wall 44 of the tissue container 40 may be made of a composite weave 234 for some embodiments. This composite weave 234 may include or otherwise be made of strands 230, 232 that include materials such as polyester, polyethylene, metal, stainless steel, or any combination of metal and plastic or polymer. For some composite weave embodiments 234, the ratio of polymer strands 232 to metal strands 230 in the composite weave 234 may, in some cases, range from about 20% to about 80%, or from about 10% to about 90% in other cases or from about 1% to about 99% in still other cases.

Some tissue container embodiments 40 may include the interior volume 42, the opening 43 and the conductive layer 47 which includes the composite weave 234 having conductive strands 230 and non-conductive strands 232. For some of these tissue container embodiments 40 the non-conductive strands 232 may include a polymer such as polyester, polyethylene, Kevlar®, Spectra®, or nylon. For some of these tissue container embodiments 40 the conductive strands 230 may include a metal such as stainless steel, nickel titanium alloy or the like. The conductive strands 230 and non-conductive strands 232 may, in some cases, have an outer transverse dimension of about 0.01 mm to about 0.5 mm. For tissue container embodiments 40 having a composite weave 234, a variety of ratios for non-conductive strands 232 to conductive strands 230 may be used. For some embodiments, the ratio of non-conductive strands 232 to conductive strands 230 may be about 10% to about 90%, more specifically, about 20% to about 80% as well as any other suitable ratios as discussed herein. Referring to FIGS. 9-11, for some embodiments, the tissue container 40 may optionally further include a second non-conductive layer 102 disposed on an inside surface 196 of the conductive layer 47, a third non-conductive layer 198 disposed on an outside surface 200 of the conductive layer 47 or both of these additional layers.

Referring to FIGS. 19-21, some embodiments of a method of containing and isolating a tissue specimen 15 within a patient's body 20 using the tissue container 40 having a configuration as discussed above, may include inserting the tissue container 40 into the body cavity 18 of the patient 20, the tissue container 40 including an interior volume 42, an opening 43 and a conductive layer 47 which comprises a composite weave 234 including conductive strands 230 and non-conductive strands 232. The tissue specimen 15 may then be inserted through the opening 43 of the tissue container 40 and into the interior volume 42 of the tissue container 40 and an entire edge of the opening 43 withdrawn from within the body cavity 18 to a position outside the patient's body 20.

Embodiments of the contact detection system 70 of tissue containment and removal system embodiments 10 may also be coupled to various instruments other than the bulk tissue reducer 30 that might come in contact or into close proximity with the conductive element 46 of the tissue container 40. Exemplary embodiments of such instruments may include the tenaculum 106, atraumatic grasper 224, camera 226, Lahey tenaculum, ring forceps, speculum, vaginal trocar, needle, or any other object that could come in contact with the conductive layer 47 of the tissue container 40. Any of these types of instruments may be configured such that if the conductive element 46 of the conductive container layer 47 is contacted or approximated by any of these types of instruments an alert can be sent to the user and/or the power to the motor 51 of the tissue cutter of the bulk tissue reducer terminated.

Referring again to FIG. 3, an optional conductive tenaculum conduit 236 is shown that serves to electrically couple the tenaculum 106 to the contact detection system 70 and detection circuit 75 thereof in a manner similar to the coupling of the tissue cutting blade 36 of the bulk tissue reducer 30 to the contact detection system 70. As such, FIG. 3 also shows a contact detection system embodiment 70 that is operatively coupled between a surgical instrument in the form of the tenaculum embodiment 106 and the conductive element 46 of the tissue container embodiment 40. Such contact detection system embodiments 70 may thus detect when the tenaculum 106, or any other suitable coupled surgical instrument, breaks through the non-conducive layer 102 and comes into contact or close proximity with the conducive element 46 of the tissue container 40. This configuration may be used to alert the user that the tissue container 40 has been breached, compromised or is about to be contacted by the tenaculum 106 or any other suitably configured instrument that may be useful when disposed within the interior volume 42 of the tissue container 40 during a tissue removal procedure.

Some embodiments of such a tissue containment and removal system 10 may include a tissue container 40 having a conductive layer 47 which includes a conductive element 46, an interior volume 42 and an opening 43. The tissue containment and removal system 10 may also include a surgical instrument which is configured for use within the interior volume of the tissue container 40 and which includes a conductive portion 106' which for the stainless steel tenaculum 106, comprises the entire instrument 106. The system 10 may further include the contact detection system 70 having a detection circuit 75 that is operatively coupled to the conductive portion 106' and the conductive element 46 of the tissue container 40. The detection circuit 75 may be configured to generate the continuity signal 76 between the conductive portion 106' and the conductive element 46 and measure the impedance value 77 between the conductive portion 106' and the conductive element 46. The contact detection system 70 may also include the controller 80 which is configured to actuate and emit a warning signal whenever the impedance 77 between the conductive portion and the conductive element is at or below a predetermined impedance threshold value. For some such embodiment, the surgical instrument may include the tenaculum 106 having a body portion made from metal that comprises the conductive portion 106'.

Some such tissue containment and removal system embodiments 10 may include an audible signal emitter 190. For such embodiments, the controller 80 may be configured to actuate an audible warning signal from the audible signal emitter 190 whenever the impedance 77 between the conductive portion 106' and the conductive element 46 of the tissue container 40 is at or below the predetermined impedance threshold value. In addition, some such tissue containment and removal system embodiments 10 may include a visual signal emitter 190. For such embodiments, the controller 80 may be configured to actuate a visual warning signal from the visual signal emitter 190 whenever the impedance 77 between the conductive portion 106' and the conductive element 46 is at or below the predetermined impedance threshold value.

In some instances, the contact detection system may have an instrument terminal 238, as shown in FIG. 13, that is operatively coupled to the controller 80 and to the conductive portion 106' with the conductive tenaculum conduit 236, and the container terminal 137 that is operatively coupled to the controller 80 and to the conductive element 46 of the tissue container 40 with a conductive conduit 72. In addition the conductive conduit 236 that operatively couples the instrument terminal 238 and conductive portion 106' may include a snap connector 240 that is configured to provide a releasable electrical coupling and the conductive conduit that operatively couples the container terminal 137 and conductive element 46 of the tissue container 40 may include the snap connector 104 that is configured to provide a releasable electrical coupling.

The contact detection system 70 for this type of tissue containment and removal system embodiment may have the same features, dimensions and materials as those of the contact detection system 70 of the tissue containment and removal system embodiment 10 discussed above with regard to monitoring contact of the conductive element 46 by the bulk tissue reducer 30 as shown in FIGS. 8 and 13. More specifically, the controller 80 may have a console printed circuit board 82 and a motor driver 84 operatively coupled to the motor 51. The controller 80 may further have a signal generator 120, a processor 86 and a memory 88 operatively coupled to the processor 86. The contact detection system 70 may include a power supply 112 operatively coupled to the controller 80.

In addition, for these same tissue containment and removal system embodiments 10 that monitor the impedance value 77 between the surgical instrument including the tenaculum 106 and the conductive element 46, the tissue container 40 may include the second non-conductive layer 102 disposed on the inside surface 196 of the conductive layer 47 as seen in FIGS. 9 and 10. The tissue container 40 may also additionally include the third non-conductive layer 198 disposed on the outside surface 200 of the conductive layer 47. The conductive layer 47 disposed between the second and third layers 102, 198 may include the composite weave 234 having non-conductive strands 232 interwoven with conductive strands 230 which comprise the conductive element 46 for such a container embodiment 40. For some embodiments, the conductive layer 47 disposed between the second and third layers 102, 198 may also include the thin flexible layer of non-conductive polymer material with a pattern of conductive ink 95 which may have a flexible configuration, which may be printed onto an outside surface thereof and which may serve as the conductive element 46 of such a tissue container embodiment 40 as seen in FIG. 14. Referring again to FIG. 12, for some tissue container embodiments 40, the conductive layer 47 and conductive element 46 thereof disposed between the second layer 102 and the third layer 198 may include a wire mesh 100 with all of the strands of the wire mesh 100 including conductive metal strands 230. The wire mesh 100 may include or be made from stainless steel in some instances.

Referring to FIGS. 16-18, the tissue cutter embodiment 34 that may be used with system embodiments 10 that monitor the impedance value 77 between the surgical instrument including the tenaculum 106 and the conductive element 46 includes the elongate tube 202 having the inner lumen 37 extending a length thereof. For some embodiments, the tissue cutting blade 36 includes the sharpened distal end of the elongate tube 202 of the tissue cutter 34 that has the beveled configuration, although alternative configurations may be used. For the embodiment shown, the shape of the tissue cutting blade 36 is circular in a transverse section profile with the entire tissue cutting blade 36 lying in a plane which is perpendicular to the longitudinal axis 204 of the elongate tube 202 of the tissue cutter 34. The elongate tube 202 of the tissue cutter 34 may be made from any suitable high strength material that may also optionally be conductive. Metals such as nickel titanium alloy, stainless steel and the like may be used in some cases. For the illustrated embodiment, the bulk tissue reducer 30 includes the housing 206 which is operatively coupled to the tissue cutter 34 and a cannula 38 which is secured to the housing 206. The cannula embodiment 38 may have an elongate hollow configuration and include the inner lumen 39 that extends the length thereof. As shown, the cannula 38 is disposed over the elongate tube 202 of the tissue cutter 34 with a close fit between the outside surface 208 of the elongate tube 38 and the inside surface 210 of the cannula 38.

Also, as discussed above with regard to the particulars of exemplary detection circuit embodiments 75 that may be used with system embodiments 10 that monitor the impedance value 77 between the surgical instrument including the tenaculum 106 and the conductive element 46, the proximity value 108' (not shown) of the conductive portion 106' of the tenaculum 106 with respect to the conductive element 46 of the tissue container 40 may be represented by various corresponding measured parameters such as impedance 77, measured current flow of the continuity signal 76, Vout signal from the Vout terminal 110 etc. For some embodiments, the impedance threshold value may be selected to correspond to a proximity value 108', which indicates the distance of separation between the conductive portion 106' and the conductive element 46, of up to about 1 mm. In order to provide a clinically safe continuity signal, in some cases, the detection circuit 75 may be configured to generate a continuity signal 76 including an alternating current having a frequency of about 10 kHz to about 30 kHz, more specifically, about 20 kHz, a maximum current flow of up to about 10 mA, a square wave continuity signal or any suitable combination of these parameters.

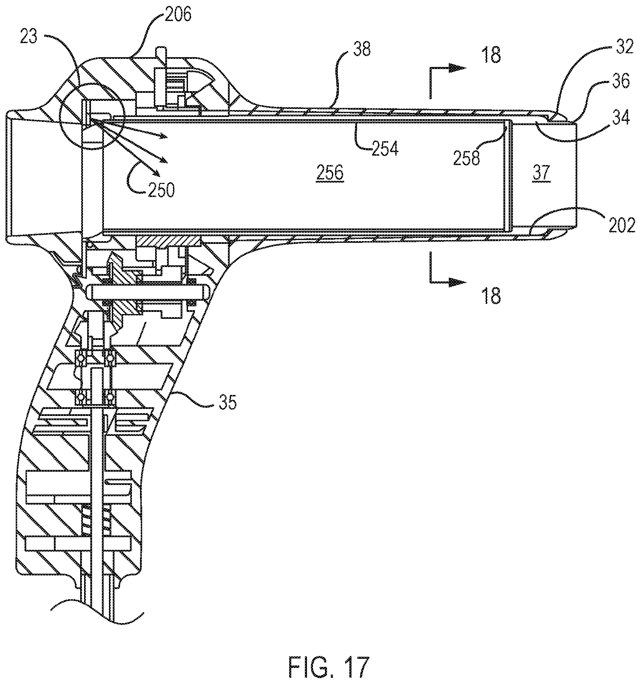

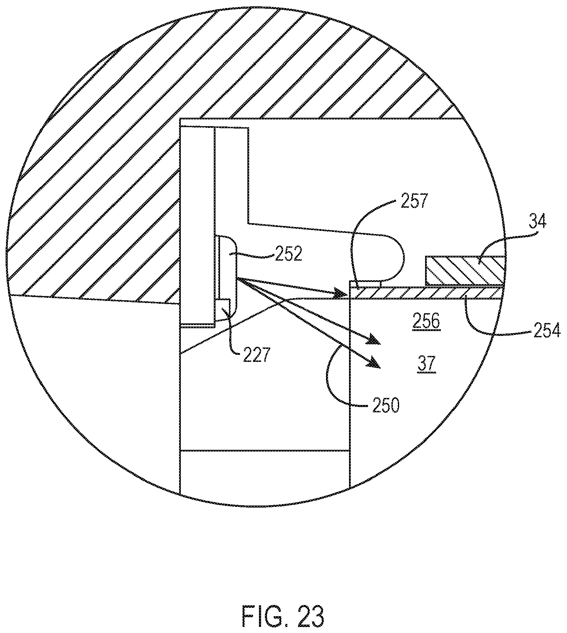

As discussed briefly above, during morcellation or reduction of a tissue specimen 15 within an interior volume 42 of the tissue container 40, it may be useful in some cases to maintain alignment of the bulk tissue remover 30 with the tissue specimen 15. In order to maintain such alignment, light energy 250 emitted from a light energy source 252 shining down the bore 37 of the tissue cutter 34 of the bulk tissue reducer 30, as shown in FIGS. 17 and 23, may be visualized through the wall 44 of the tissue container 40 even if the tissue container 40 is partially opaque and not entirely transparent. For such embodiments, if the wall structure 44 of the tissue container 40 is at least translucent, the visualization of light energy being emitted from gaps disposed between a distal end 33 of the tissue cutter 34 of the bulk tissue reducer 30 and the tissue specimen 15 itself may serve as an indication of certain types of misalignment between the tissue cutter 34 and the tissue specimen 15.

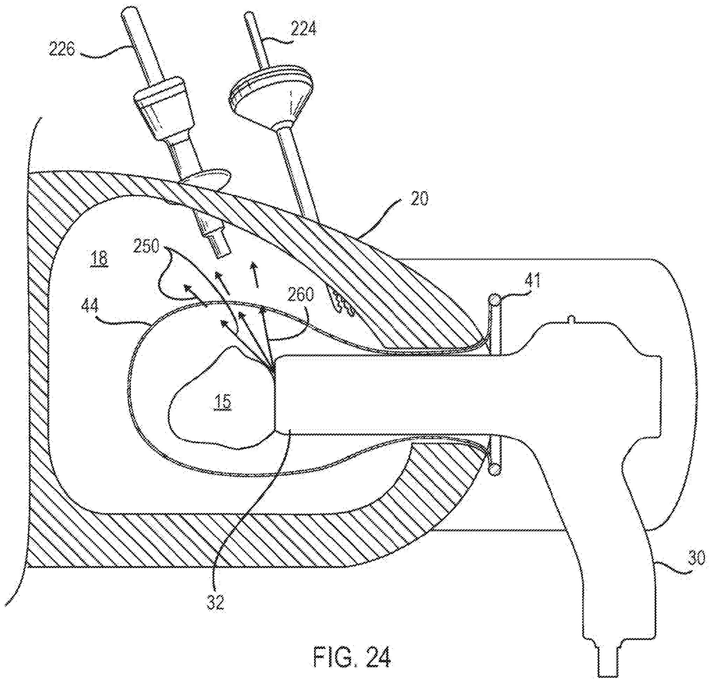

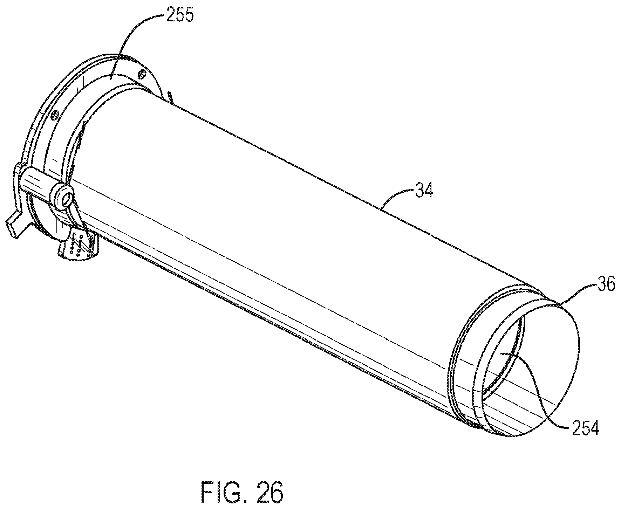

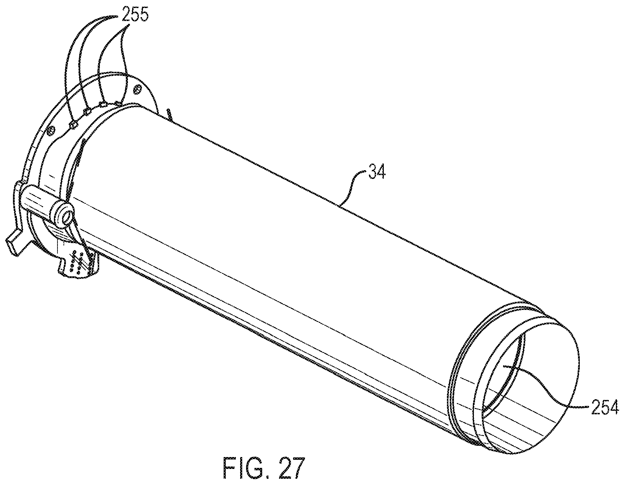

FIGS. 17, 23 and 24 show how a light energy source 252 or a camera 227 disposed adjacent an edge of the bulk tissue reducer 30 itself may be used to shine or view down the bore 37 of the tissue cutter 34 of the bulk tissue reducer 30. In some cases, the coupling of the light energy 252 into the bore 37 or the light guide 254 may be enhanced by a light cone 255 which is conical structure disposed over the light energy sources 252 as seen in FIG. 26 and which has been removed in FIG. 27 for purposes of illustration. The light energy source 252 or camera 227 may be positioned so as to not interfere with the extraction of the tissue specimen 15 and operation of the tenaculum 106 (as shown in FIG. 22) that is being used to extract the tissue specimen 15 through the bore 37. FIG. 24 shows how the light energy 250 transmitted through the wall 44 of the tissue container 40 and observed or monitored by the camera 226 disposed in the body cavity 18 of the patient 20 may be an indicator of whether the tissue specimen 15 that is intended to be reduced or morcellated is firmly and fully in contact with all 360 degrees of the tissue cutting blade 36 of the bulk tissue reducer 30. For some embodiments, the light energy 250 may include a colored light energy such as a red light energy. Any leaking of this colored light energy 250 into the interior volume 42 of the tissue container 40 may be identified by the camera 226 through the wall 44 of the tissue container 40 as an indicator that there is not full tissue contact between the tissue cutting blade 36 of the tissue cutter 34 and the tissue specimen 15. The tissue container 40 may be designed such that the light energy 250 transmits through the container 40 and the wall structure 44 thereof is either translucent, transparent or otherwise not fully opaque. The light energy 250 may also include white light energy, or any other type of electromagnetic energy either within or outside of the visible light energy spectrum, such as infrared, near infrared, ultraviolet, radio waves, microwaves, x-rays, etc.

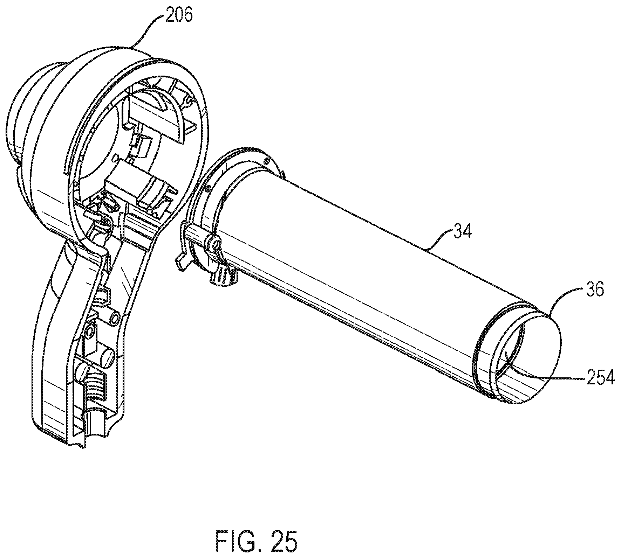

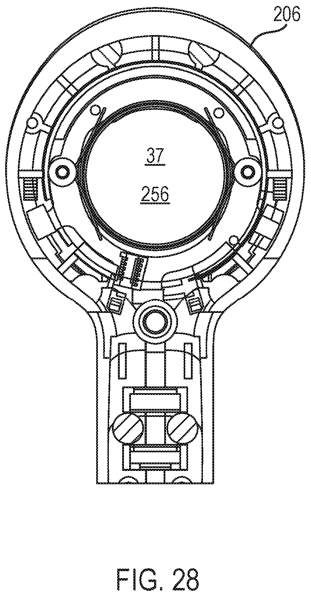

As shown in FIGS. 25-28, light energy 250 and light energy sources 252 thereof may be incorporated into the housing 206 of the bulk tissue reducer 30 to aid in visualization of the tissue specimen 15 through the bore 37 of the tissue cutter 34 of the bulk tissue reducer 30. In some cases, the light energy 250 may be directed down the handpiece 35 and/or housing 206 of the bulk tissue reducer 30.

Referring again to FIGS. 17, 23, and 24-28, some embodiments of the tissue containment and removal system 10 may include the tissue container 40 having a translucent wall structure 44 and a bulk tissue reducer 30. The bulk tissue reducer embodiment 30 may include the tissue cutter 34 having a hollow structure with the inner lumen 37 extending a length thereof and the tissue cutting blade 36 disposed at a distal end 33 of the tissue cutter 34. The bulk tissue reducer 30 may also include a light energy source 252 configured to emit light energy 250 in a distal direction through the inner lumen 37 and from the distal end 33 of the tissue cutter 34. For some embodiments, the bulk tissue reducer 30 may further include the housing 206 that is secured in fixed relation with an optional light guide 254. Generally, for such embodiments, the tissue cutter 34 is operatively coupled to the housing 206 so as to allow rotation of the tissue cutter 34 about a longitudinal axis 204 (see FIG. 16) thereof with respect to the housing 206. In some instances, the translucent wall structure 44 of the tissue container 40 may include a thin layer 102 of polymer material including polyester, polyethylene, polyurethane, polypropylene, PET, PETG, aramid and para-aramids, poly-paraphenylene terepthalamide (KEVLAR®), and aliphatic or semi-aromatic polyamides (NYLON®). The wall 44 of the tissue container 40 used for this embodiment may also use any other suitable materials or construction including the conductive element embodiments 46 discussed herein.

For some such system embodiments 10, the light energy source 252, which may include LED light sources 252 or the like, may be disposed adjacent a proximal end of the inner lumen 37 of the tissue cutter 34 within the housing 206. In some cases, the bulk tissue reducer 30 may further include the optional light guide 254 which is operatively coupled to the light energy source or sources 252 such that at least some of the light energy 250 emitted from the light energy sources 252 is transmitted into and transmitted or conducted by the light guide 254. The light guide 254 may be disposed within the inner lumen 37 of the tissue cutter 34 and be configured to transmit light energy 250 from the light energy source 252 in distal direction through the light guide 254 to be emitted out of the distal end 33 of the tissue cutter 34. For some embodiments, the light guide 254 may include an elongate hollow configuration having an inner lumen 256 extending a length thereof. Embodiments of the optional light guide 254 may include a translucent polymer material that is configured to transmit the light energy 250 from a proximal end 257 of the light guide 254 to a distal end 258 of the light guide 254. In some cases, the translucent polymer material of the light guide 254 may include polycarbonate. In addition, in some cases, the inner surface of the light guide 254 may reflective so as to promote partial or total internal reflection of light energy 250 propagating within the inner lumen of the light guide 254 is a distal direction. For such embodiments, the light guide 254 may be made from a material that is not translucent, including metals such as stainless steel or the like. As mentioned above, the tissue cutter 34 is configured to rotate with respect to the housing 206, however, the elongate hollow structure of the light guide 254 may be secured to the housing 206 so as to remain stationary with respect to the housing 206 as the tissue cutter 34 rotates about the longitudinal axis 204 thereof during actuation of the tissue cutter 34.

As discussed above, in some cases, the light energy source 252 may include a plurality of light energy sources 252 disposed at the proximal end of the inner lumen 37 of the tissue cutter 34 and operatively coupled to the light guide 254 as shown in FIG. 23. Some embodiments of the plurality of light energy sources 252 may include light emitting diodes that may emit light energy in any suitable light wavelength. In some cases, the light emitting diodes may be red light emitting diodes configured to emit red light. Typically, the light energy source(s) 252 will be configured to emit light energy 250 which is bright enough to be visible through the translucent wall structure 44 of the tissue container 40. Some bulk tissue reducer embodiments 30 may include 1, 2, 3, 4, 5 or more such light energy sources 252.

Some embodiments of a method of containing and removing a tissue specimen 15 from the patient's body 20 may include inserting the tissue container 40 into the body cavity 18 of the patient 20, inserting the tissue specimen 15 through the opening 43 of the tissue container 40 and into the interior volume 42 of the tissue container and withdrawing the entire edge of the opening 43 of the tissue container 40 from within the body cavity 18 to a position outside the patient's body 20 as shown in FIGS. 19-21. This method may also include inserting the distal end 32 of the bulk tissue reducer 30 into the interior volume 42 of the tissue container 40 until the tissue cutting blade 36 of the tissue cutter 34 of the bulk tissue reducer 30 contacts the tissue specimen 15. Light energy 250 may then be emitted from the distal end 33 of the tissue cutter 34 in generally a distal direction towards the tissue specimen 15 in contact with the tissue cutting blade 36. Although the light energy 250 emitted from the distal end 33 of the tissue cutter 34 is being transmitted in a generally distal direction, the light energy 250 is launched into the inner lumen 37 of the tissue cutter 34 and wall structure of the hollow tubular light guide 254 at a variety of angles and thus by the time the light energy 250 is emitted from the distal end 33 of the tissue cutter 34, it is being emitted in a wide variety of angles forming a very broad solid angle of emission. Leakage of light energy 250 may then be observed from between the distal end 33 of the tissue cutter and distal end 32 of the bulk tissue reducer 30 and the tissue specimen 15 as shown in FIG. 24. The intensity and orientation of the light energy leakage 260 observed by a user through camera 226 or any other suitable instrument may be used for manipulating the alignment between the distal end 32 of the bulk tissue reducer 30 and the tissue specimen 15 to minimize the amount of light energy leakage 260 between the distal end 32 of the bulk tissue reducer 30 and the tissue specimen 15.

In some cases, the method may further include actuating the tissue cutter 34 of the bulk tissue reducer 30 and deactivating the tissue cutter 34 of the bulk tissue reducer 30 upon observation of light energy leakage 260 from between the distal end 32 of the bulk tissue reducer 30 and the tissue specimen 15. In some cases, the method may further include actuating the tissue cutter 34 of the bulk tissue reducer 30, contacting the tissue specimen 15 with the tissue cutting blade 36 of the tissue cutter 34 and reducing the tissue specimen 15 with the actuated tissue cutter 36. In some instances, the method may further include securing a distal end 107 of a tenaculum 106 to the tissue specimen 15 and pulling a reduced portion of the tissue specimen 15 through an inner lumen 37 of the tissue cutter 34 while reducing the tissue specimen 15 with the actuated tissue cutter 34 until at least a portion of the tissue specimen 15 is disposed outside of the bulk tissue reducer 30 and the patient's body 20 as shown in FIG. 22.

As discussed above, in order to contain and isolate a tissue specimen 15 prior to reduction or morcellation of the tissue specimen 15, it may be desirable to reliably and consistently deploy a suitable tissue container embodiment 40 around the tissue specimen 15 in some cases, as shown in FIGS. 19-21. This process may typically be carried out in the confined space or cavity 18 within the patient's body 20. In some cases, this process may be facilitated by the use of a suitable container deployer assembly 270 or container deployer 272 thereof. In some cases, such container deployer assembly embodiments 270 may be configured to perform deployment of a tissue container 40 while maintaining some control of the orientation of the tissue container 40 during deployment. Some embodiments of such container deployer assemblies 270 may be configured to operate in a manner similar to that of a rivet gun. This type of configuration may be actuated using a spring loaded mechanism, compressed air, or other mechanical or electrical actuator to deploy the container from a sheath of the tissue container deployer assembly (not shown).

Referring to FIGS. 30-31, in some instances, such a container deployer assembly 270 may include a sheath 274 that may have an elongate hollow configuration made from a rigid polymer or other suitable material. The sheath 274 may be placed through the skin of the patient 20 in order to gain access to an interior portion 18 of a patient's body 20. The sheath 274 may have a size which corresponds to a size of a corresponding bulk tissue reducer 30, or to a size of a common laparoscopic trocar incision lengths e.g. trocar incision lengths of about 5mm, about 8mm, about 10mm, about 12mm or other suitable lengths. Additionally, the container deployer assembly embodiments 270 may be used to place the sheath 274 through a natural body orifice such as rectum or vagina 24.

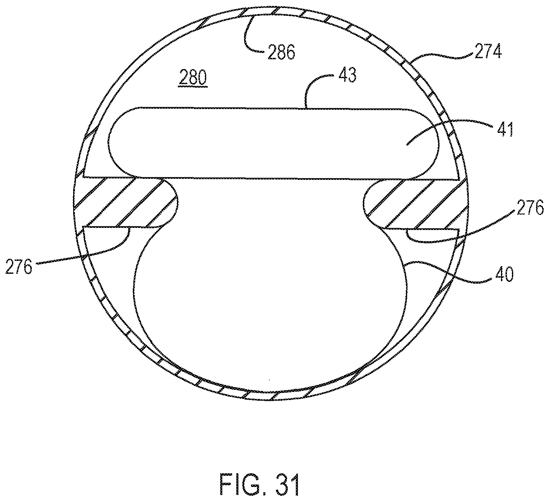

FIGS. 29-32 show a container deployer assembly embodiment 270 for a tissue container 40 that may be configured for use with many different procedures. One possible use may include placing a tissue container 40 within the abdominal cavity 18 of a patient 20 for the purpose of capturing and isolating a tissue specimen 15 such as the uterus of a patient 20 for the purpose of performing a hysterectomy. The container deployer assembly embodiment 270 shown in FIGS. 29-32 may generally be configured to fit into the vaginal canal 24 and to protrude either just before or past the vaginal cuff after the uterus has been detached from the vagina 24 via colpotomy. Such container deployer assembly embodiments 270 may have features to facilitate control the orientation of the tissue container 40 during deployment. Exemplary features that may be used to provide such control may include stabilizer ridges (guide rails) 276 or other asymmetric features as shown in FIG. 31 to engage the tissue container 40 and ensure that the tissue container 40 is placed on the floor of the abdomen which may be useful for some tissue removal procedures. In some instances, the tissue container 40 may be deployed into a position that is disposed between the uterus and the floor of the abdomen.

FIG. 30 shows a container deployer assembly embodiment 270 that includes a pusher rod 278 that may be used to push the tissue container 40 out of an inner lumen 280 the sheath 274 to aid in the deployment of the tissue container 40 into the abdomen or other body cavity 18 of the patient 20. Referring to FIG. 30, a tether 282 is shown extending from the tissue container 40 to a position disposed outside of the sheath 274. It may also be possible to have the tether 282 disposed on the inside of the sheath 274, as shown in FIG. 30. It may also be possible for the pusher rod 278 to include an elongate notch or groove 284 extending longitudinally along an outer surface thereof for the tether 282 to fit between the pusher rod 278 and the sheath 274 in in the event that an inside surface 286 of the sheath 274 and an outside surface 288 of the pusher rod 278 have a tight fit therebetween. For some embodiment, the tether 282 may also include a conductive conduit and serve as the container conduit 72 which is in electrical communication with the conductive element 46 of the container 40. As such, the tether 282 may, in some cases, include a snap connector 104 that may be configured to releasably and operatively couple to the container terminal 137 of the detection circuit 75.

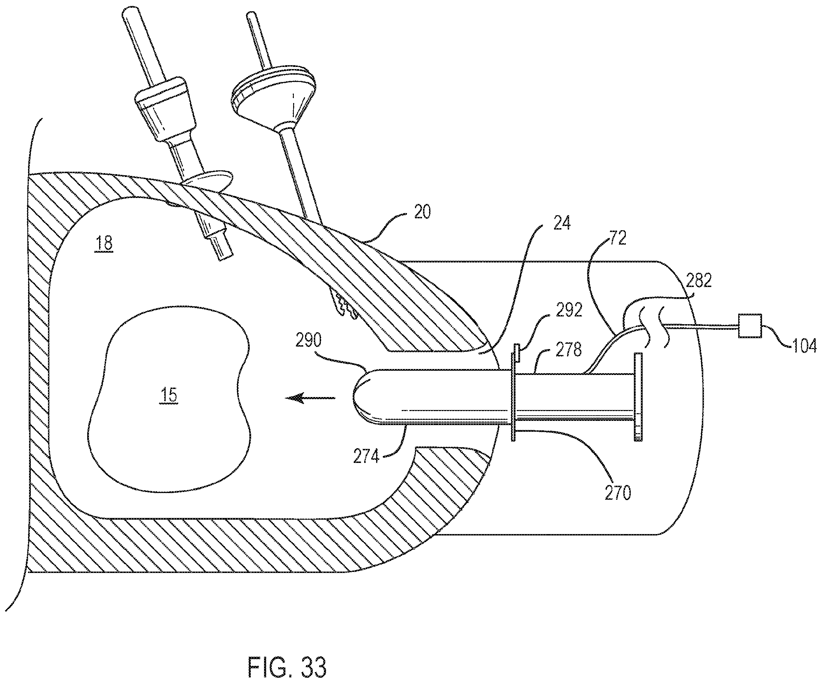

Some embodiments of the container deployer assembly 270 include the container deployer 272 and the tissue container 40 disposed therein and ready to be deployed. Embodiments of the container deployer 272 may include the sheath 274 and the pusher rod 278 which is configured to axially slide within the inner lumen 280 of the sheath 274 in order to deploy the tissue container 40. Some sheath embodiments 274 may be shaped such that they include a rounded atraumatic distal tip 290 so that they may be easily introduced into the vagina, rectum, port, or other natural orifice 24 or surgically created orifice without trauma to surrounding tissue. Such container deployer assembly 270 embodiments may have many uses including placement of the tissue container 40 into the abdominal cavity 18 for the purposes of capturing the uterus for a hysterectomy, when a woman's uterus needs to be removed. The container deployer assembly embodiment 270 is shown in FIG. 33 being inserted into the vagina 24 of the patient 20 as indicated by the arrow 292 for the purposes of containing and removing the patient's uterus 15 for performing a hysterectomy. FIG. 33 shows the sheath 274 and tissue container 40 of the container deployer assembly 270 being introduced into the vagina 24 with the tissue container 40 already pre-loaded into the sheath embodiment 274. In some instances, during such a procedure, the uterus 15 may be detached from the vagina via colpotomy and the sheath 274 may be inserted up to or past the vaginal cuff as shown.

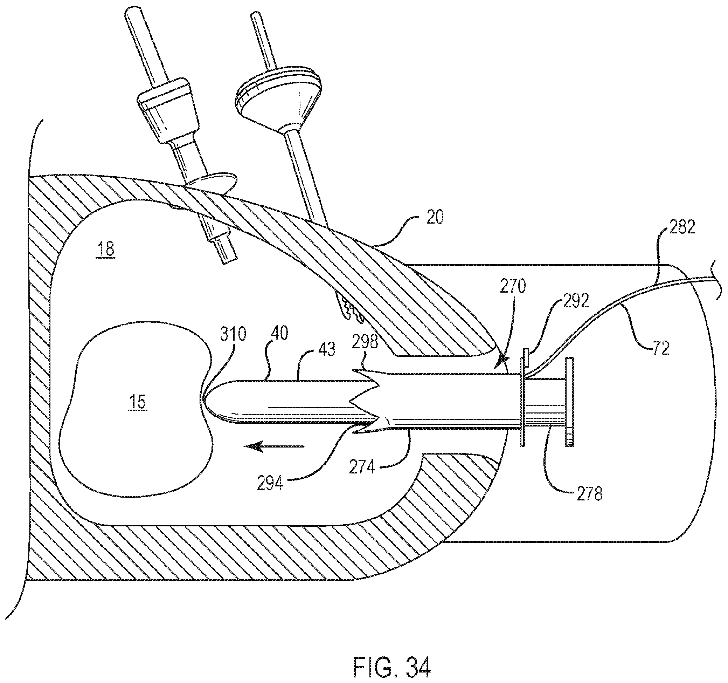

Once the sheath 274 and tissue container disposed therein are optimally situated in the vagina 24, the pusher rod 278 may be pushed in a distal direction relative to the sheath 274 which may be used to effectively deploy the tissue container 40 out of the distal end 290 of the sheath 274 and into the cavity 18 within the abdomen or pelvis of the patient as shown in FIG. 34. FIG. 33 shows the tether 282 extending from the tissue container 40 to a position outside of the sheath 274. It is also possible for some portions of the tether 282 to be disposed on the inside of the sheath 274, as shown in FIG. 30 and for the pusher rod 278 to have the notch or groove 284 to accommodate the tether 282 below a nominal outer surface 288 of the pusher rod 282 in the event that the sheath 274 and the pusher rod 278 have a tight fit therebetween.

Some sheath embodiments 274 may include features shown in FIG. 31 to ensure that the orientation, such as the circumferential orientation, of the tissue container 40 may be controlled during deployment of the tissue container 40. Such features may include stabilizer ridges 276 extending inwardly from an inside surface 286 of the inner lumen 280 of the sheath 274 or other symmetric or asymmetric features as shown in FIG. 31. Such features 276 may be useful to ensure that the tissue container 40 is placed on the floor of the abdomen when being deployed through the vagina 24 for containment and removal of a tissue specimen 15. In some cases, the tissue container 40 may be deployed between the uterus and the floor of the abdomen. Some sheath embodiments 274 may also have an orientation indicator 292, such as circumferential orientation, as shown in FIGS. 29, 30 and 32 so that a user may be made aware which orientation the opening 43 of the tissue container 40 is disposed in once the tissue container 40 of such an embodiment is disposed at a desired location within a patient's body 20 and ready to be deployed. For example, if the orientation indicator 292 is pointing towards an operating room ceiling during insertion and subsequent deployment, then the opening 43 of the tissue container 40 will also point in this direction once deployed, as shown in FIG. 34, for some embodiments.

For some embodiments, the tissue container 40 may be partially deployed out of the sheath 274 such that an opening 294 at the distal end 290 of the sheath 274 may be configured to spring open like a hoop but with a portion of the hoop remaining inside the sheath 274. For such embodiments, full deployment may be prevented by holding a little tension on a tether 282. In some instances, the rim 41 of the container 40 may be attached to a rigid member (not shown) which is used to control the hoop or opening 43 of the rim 41. The rigid member can be a rigid wand in both a hand-held and robot-controlled embodiment. The rigid member guiding or as a part of the tissue container 40, could also be constructed to be made to be a part of the container deployer 272. In effect, an operator of embodiments of the container deployer 272 may have the open tissue container 40 on a rigid control wand and the operator may work as a team with the laparoscopic surgeon to effect containment of the target tissue specimen 15 within the interior volume 42 of the tissue container 40.

Some embodiments of the tissue container deployer assembly 270 may include a tissue container deployer 272 having the sheath 274 with the inner lumen 280, the rounded distal tip 290 including longitudinal slits 296 that converge together and which form petals 298 in the distal tip 290 of the sheath 274 which are configured to open upon the application of distal axial pressure from within the inner lumen 280. The tissue container deployer 272 may also include the pusher rod 278 that has an elongate configuration with the outside surface 288 which is sized to fit and translate axially within the inner lumen 280 of the sheath 274 and which has an axial length equal to or larger than an axial length of the inner lumen 280 of the sheath 274. The tissue container embodiment 40 is disposed within the inner lumen 280 of the sheath 274 in contracted state, the tissue container 40 including a wall 44 having a thin flexible configuration, an interior volume 42 and an opening 43 in communication with the interior volume 42.

In some cases, embodiments of the sheath 274 may further include the plurality of stabilizer ridges 276 which are each secured to the inner lumen 280 and extend radially inwardly from an inside surface 286 of the inner lumen 280 and which each have an elongate configuration with a longitudinal axis that is substantially parallel to a longitudinal axis 300 of the sheath 274 and pusher rod 278. In some instances, the opening 43 of the tissue container 40 includes the rim 41 disposed about the opening 43 that engages the tissue stabilizer ridges 276 of the sheath 274 so as to prevent rotation of the tissue container 40 within the inner lumen 280 of the sheath 274 and positions the tissue container 40 with the opening 43 thereof facing a fixed and known circumferential orientation. For some embodiments, the rim 41 of the tissue container 40 may have a resilient configuration that opens when in an unconstrained state.

For some embodiments, the plurality of stabilizer ridges 276 may be evenly spaced about the inner lumen 280 in a circumferential orientation and the number of stabilizer ridges 276 may include 2, 3, 4 or more stabilizer ridges 276. In some cases, the stabilizer ridges 276 may have a longitudinal length that is at least twice a transverse outer dimension of the sheath 274 and may extend radially inwardly about 0.05 inches to about 0.4 inches from the inside surface 286 of the inner lumen 280.

Some sheath embodiments 274 may be made from a polymer material which may include ABS plastic, polycarbonate, PEEK or PVC. Some sheath embodiments 274 may have an axial length of about 15 cm to about 35 cm, a transverse dimension of about 0.4 inches to about 1.5 inches and a wall thickness of about 0.02 inches to about 0.1 inches. In some cases, such sheath embodiments 274 may further include the orientation indicator 292 that may, in some circumstances, be used to indicate the circumferential orientation of the opening 43 of the tissue container 40 to a user of the container deployer assembly 270. Some sheath embodiments 274 may further include a flange 302 disposed on a proximal end thereof with the orientation indicator 292 including an arrow shaped body secured to the flange 302 with the arrow 304 pointing in a direction which is the same as the direction that the opening 43 of the tissue container 40 is facing when disposed within the sheath 274.

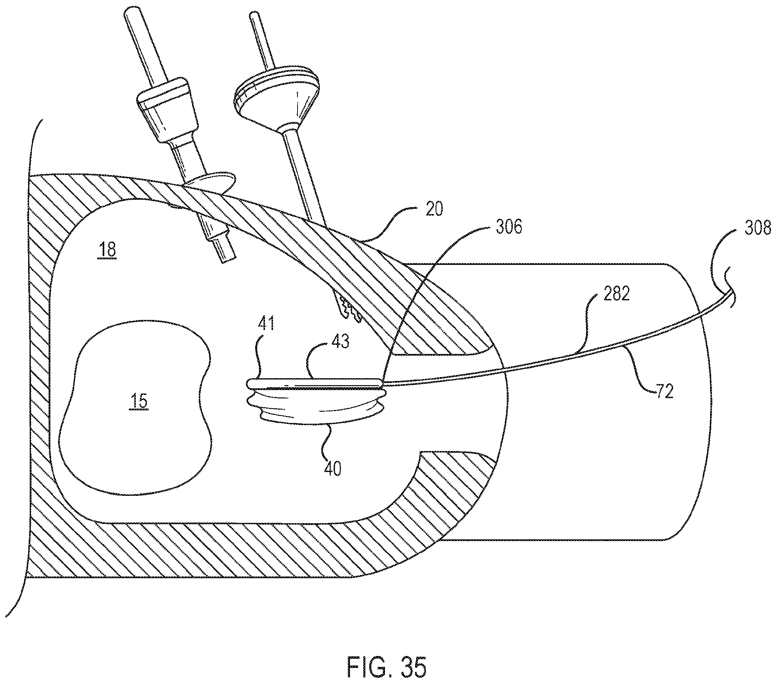

Some embodiments 270 may include the tether 282 that has a thin flexible configuration and a distal end 306, as shown in FIG. 35, which is secured to the rim 41 disposed about the opening 43 of the tissue container 40 and a proximal end 308 that extends out of the inner lumen 280 of the sheath 274 prior to deployment. FIG. 35 shows the tissue container 40 fully ejected from the distal port 294 of the sheath 274 and disposed within the body cavity 18 with the tether 282 extending from the cavity 18 to a position outside the patient's body 20. In some instances, for such embodiments, the pusher rod 278 may include the longitudinal groove 284 disposed along an outer surface 288 thereof with the tether 282 disposed within the longitudinal groove 284 between an outer surface of the longitudinal groove and an inside surface 286 of the sheath 274.

Some method embodiments of deploying the tissue container 40 may include inserting the distal end 290 of the sheath 274 of a tissue container deployer assembly 270 through the body opening 24 and into a desired position within an interior cavity 18 of the patient 20 as shown in FIG 33. The pusher rod 278 of the tissue container deployer assembly 270 is axially advanced in a distal direction with respect to the sheath 274 while simultaneously axially advancing the tissue container 40 in the contracted state disposed within the inner lumen 280 of the sheath 274 as shown in FIG. 34. The tissue container 40 is so axially advanced with the distal end of the pusher rod 278 which abuts a proximal end of the tissue container 40. As the pusher rod 278 and tissue container 40 are axially advanced, the method also includes opening flexible petals 298 formed by longitudinal slits 296 in the distal end 290 of the sheath 274 with a distal end 310 of the tissue container 40 as constituted in the contracted state to form the distal port or opening 294 in the sheath 274 for distal ejection of the tissue container 40 from the inner lumen 280 of the sheath 274. The method further includes continuing to axially advance the tissue container 40 with the pusher rod 278 until the tissue container 40 is fully ejected from the distal port 294 of the sheath 274 and into the interior cavity 18 of the patient 20 as shown in FIG. 35.

As discussed above, the sheath 274 may include a plurality of stabilizer ridges 276 and some method embodiments include stabilizing the circumferential orientation of the tissue container 40 with the stabilizer ridges 276 during axial advancement of the tissue container 40 with the pusher rod 276. In some cases, the method may also include proximally withdrawing the rim 41 of the of the tissue container 40 from the interior cavity 18 of the patient 20 and out of the body opening 24 to a position outside the patient's body 20 as shown in FIG. 21. For some embodiments, the method may also include proximally withdrawing the rim 41 of the tissue container 40 from within the body cavity 18 of the patient 20 to a position outside the patient's body 20 with the tether 282.

Features described herein with respect to different methods of use or different features, instruments, components, or their order of use may interchangeably be used among the various methods. The presence or absence of a particular step or component should not be construed as limiting the methods described herein,

With regard to the above detailed description, like reference numerals used therein may refer to like elements that may have the same or similar dimensions, materials and configurations. While particular forms of embodiments have been illustrated and described, it will be apparent that various modifications can be made without departing from the scope of the present invention as defined by the appended claims.

Accordingly, it is not intended that the invention be limited by the foregoing detailed description.

Modifications may be made to the foregoing embodiments without departing from the basic aspects of the technology. Although the technology may have been described in substantial detail with reference to one or more specific embodiments, changes may be made to the embodiments specifically disclosed in this application, yet these modifications and improvements are within the scope as defined by the appended claims.

The technology illustratively described herein suitably may be practiced in the absence of any element(s) not specifically disclosed herein. Thus, for example, in each instance herein any of the terms "comprising," "consisting essentially of," and "consisting of" may be replaced with either of the other two terms. The terms and expressions which have been employed are used as terms of description and not of limitation, and various modifications are possible within the scope as defined by the appended claims. The term "a" or "an" may refer to one of or a plurality of the elements it modifies unless it is contextually clear either one of the elements or more than one of the elements is described. Although the present technology has been specifically disclosed by representative embodiments and optional features, modification and variation of the concepts herein disclosed may be made, and such modifications and variations may be considered within the scope as defined by the appended claims.

**Claims**

1. A tissue containment and removal system (10), comprising:

    a tissue container (40) comprising a conductive layer (47) which comprises a conductive element (46), an interior volume (42) and an opening (43);
    a bulk tissue reducer (30) including a tissue cutter (34) having a tissue cutting blade (36) that is configured to be conductive;
    a motor (51) which is operatively coupled to the tissue cutting blade (36) of the bulk tissue reducer (30); and
    a contact detection system (70) including:

        a detection circuit (75) that is operatively coupled to the tissue cutting blade (36) and the conductive element (46) and that is configured to generate a continuity signal between the tissue cutting blade (36) and the conductive element (46) and measure an impedance value between the tissue cutting blade (36) and the conductive element (46), and
        a controller (80) which is operatively coupled to the motor (51), and which is configured to discontinue actuation of the motor (51) and tissue cutting blade (36) whenever the impedance between the tissue cutting blade (36) and the conductive element (46) is at or below a predetermined impedance threshold value.

2. The tissue containment and removal system of claim 1 wherein the controller (80) is configured to actuate a warning signal whenever the impedance between the tissue cutting blade (36) and the conductive element (46) is at or below the predetermined impedance threshold value.

3. The tissue containment and removal system of claim 2 further comprising an audible signal emitter (190) and wherein the controller (80) is configured to actuate an audible warning signal from the audible signal emitter (190) whenever the impedance between the tissue cutting blade (36) and the conductive element (46) is at or below the predetermined impedance threshold value.

4. The tissue containment and removal system of claim 1 wherein the contact detection system (70) further includes a blade terminal (135) that is operatively coupled to the controller (80) and to the tissue cutting blade (36) with a conductive conduit (74), and a container terminal (137) that is operatively coupled to the controller (80) and to the conductive element (46) of the tissue container (40) with a conductive conduit (72).

5. The tissue containment and removal system of claim 1 wherein the controller (80) comprises a console printed circuit board (82) and a motor driver (84) operatively coupled to the motor (51).

6. The tissue containment and removal system of claim 5 wherein the controller (80) further comprises a signal generator (120), a processor (86) and a memory (88) operatively coupled to the processor (86).

7. The tissue containment and removal system of claim 1 wherein the contact detection system (70) further comprises a power supply (112) operatively coupled to the controller (80).

8. The tissue containment and removal system of claim 1 wherein the tissue container (40) further comprises a second non-conductive layer (102) disposed on an inside surface (196) of the conductive layer (47).

9. The tissue containment and removal system of claim 8 wherein the tissue container (40) further comprises a third non-conductive layer (198) disposed on an outside surface (200) of the conductive layer (47).

10. The tissue containment and removal system of claim 1 wherein the conductive layer (47) comprises a composite weave (234) comprising non-conductive strands (232) interwoven with conductive strands (230) which comprise the conductive element (46).

11. The tissue containment and removal system of claim 1 wherein the conductive layer (47) comprises a thin flexible layer of non-conductive polymer material and a pattern of conductive ink (95) printed onto an outside surface (200) thereof.

12. The tissue containment and removal system of claim 1 wherein the conductive layer (47) and conductive element (46) thereof comprises a wire mesh (100).

13. The tissue containment and removal system of claim 1 wherein the tissue cutter (34) comprises an elongate tube (202) having an inner lumen (37) extending a length thereof and the tissue cutting blade (36) comprises a sharpened distal end of the elongate tube (202).

14. The tissue containment and removal system of claim 1 wherein the detection circuit (75) is configured to generate a continuity signal comprising an alternating current having a frequency of about 10 kHz to about 30 kHz, and having a maximum current flow of up to about 10 mA.

15. The tissue containment and removal system of claim 1 wherein the bulk tissue reducer (30) further comprises a housing (206) which is operatively coupled to the tissue cutter (34) and a cannula (38) which is secured to the housing (206), which includes an elongate hollow configuration, and which includes an inner lumen (39) that extends a length thereof and is disposed over the tissue cutter (34).

**Patentansprüche**

1. Gewebeeindämmungs- und -entnahmesystem (10) mit:

einem Gewebebehälter (40) mit einer leitfähigen Schicht (47), der ein leitfähiges Element (46) umfasst, ein Innenvolumen (42) und eine Öffnung (43);
einem Gewebestückzerkleinerer (30) mit einem Gewebeschneider (34), der eine Gewebeschneidklinge (36) aufweist, die so ausgestaltet ist, dass sie leitfähig ist;
einem Motor (51), der funktional mit der Gewebeschneidklinge (36) des Gewebestückzerkleinerers (30) verbunden ist; und
einem Kontaktdetektionssystem (70) mit:

einer Erkennungsschaltung (75), die funktional mit der Gewebeschneidklinge (36) und dem leitfähigen Element (46) verbunden ist und so ausgestaltet ist, dass sie ein Durchgangssignal zwischen der Gewebeschneidklinge (36) und dem leitfähigen Element (46) erzeugt und einen Impedanzwert zwischen der Gewebeschneidklinge (36) und dem leitfähigen Element (46) misst, und
einer Steuereinheit (80), die funktional mit dem Motor (51) gekoppelt ist und so ausgestaltet ist, dass sie die Betätigung des Motors (51) und der Gewebeschneidklinge (36) unterbricht, wenn die Impedanz zwischen der Gewebeschneidklinge (36) und dem leitfähigen Element (46) einen vorbestimmten Impedanzschwellenwert erreicht oder unterschreitet.

2. Gewebeeindämmungs- und -entnahmesystem nach Anspruch 1, wobei die Steuereinheit (80) so ausgestaltet ist, dass sie ein Warnsignal auslöst, wenn die Impedanz zwischen der Gewebeschneidklinge (36) und dem leitfähigen Element (46) bei oder unter dem vorbestimmten Impedanzschwellenwert liegt.

3. Gewebeeindämmungs- und -entnahmesystem nach Anspruch 2, das ferner einen akustischen Signalgeber (190) umfasst, wobei die Steuereinheit (80) so ausgestaltet ist, dass sie ein akustisches Warnsignal mittels des akustischen Signalgebers (190) auslöst, wenn die Impedanz zwischen der Gewebeschneidklinge (36) und dem leitfähigen Element (46) bei oder unter dem vorbestimmten Impedanzschwellenwert liegt.

4. Gewebeeindämmungs- und -entnahmesystem nach Anspruch 1, wobei das Kontakterfassungssystem (70) ferner einen Klingenanschluss (135) umfasst, der funktional mit der Steuereinheit (80) und der Gewebeschneidklinge (36) über eine leitfähige Leitung (74) verbunden ist, und einen Behälteranschluss (137) umfasst, der funktional mit der Steuereinheit (80) und dem leitfähigen Element (46) des Gewebebehälters (40) über eine leitfähige Leitung (72) verbunden ist.

5. Gewebeeindämmungs- und -entnahmesystem nach Anspruch 1, wobei die Steuereinheit (80) eine Konsolen-Leiterplatte (82) und einen Motortreiber (84) umfasst, der funktional mit dem Motor (51) verbunden ist.

6. Gewebeeindämmungs- und -entnahmesystem nach Anspruch 5, wobei die Steuereinheit (80) ferner einen Signalgenerator (120), einen Prozessor (86) und einen Speicher (88) umfasst, der funktional mit dem Prozessor (86) verbunden ist.

7. Gewebeeindämmungs- und -entnahmesystem nach Anspruch 1, wobei das Kontakterfassungssystem (70) ferner eine Stromversorgung (112) umfasst, die funktional mit der Steuereinheit (80) gekoppelt ist.

8. Gewebeeindämmungs- und -entnahmesystem nach Anspruch 1, wobei der Gewebebehälter (40) ferner eine zweite nichtleitende Schicht (102) umfasst, die auf einer Innenfläche (196) der leitenden Schicht (47) angeordnet ist.

9. Gewebeeindämmungs- und -entnahmesystem nach Anspruch 8, wobei der Gewebebehälter (40) ferner eine dritte nichtleitende Schicht (198) umfasst, die auf einer Außenfläche (200) der leitenden Schicht (47) angeordnet ist.

10. Gewebeeindämmungs- und -entnahmesystem nach Anspruch 1, wobei die leitfähige Schicht (47) ein Verbundgewebe (234) umfasst, das nichtleitende Stränge (232) umfasst, die mit leitfähigen Strängen (230) verwoben sind, die das leitfähige Element (46) umfassen.

11. Gewebeeindämmungs- und -entnahmesystem nach Anspruch 1, wobei die leitfähige Schicht (47) eine dünne flexible Schicht aus nichtleitendem Polymermaterial und ein Muster aus leitfähiger Tinte (95) umfasst, das auf eine Außenfläche (200) davon gedruckt ist.

12. Gewebeeindämmungs- und -entnahmesystem nach Anspruch 1, wobei die leitfähige Schicht (47) und dessen leitfähiges Element (46) ein Drahtgeflecht (100) umfassen.

13. Gewebeeindämmungs- und -entnahmesystem nach Anspruch 1, wobei der Gewebeschneider (34) ein längliches Rohr (202) mit einem inneren Lumen (37) umfasst, das sich über dessen Länge erstreckt, und die Gewebeschneidklinge (36) ein geschärftes distales Ende des länglichen Rohrs (202) umfasst.

14. Gewebeeindämmungs- und -entnahmesystem nach Anspruch 1, wobei die Detektionsschaltung (75) so ausgestaltet ist, dass sie ein Kontinuitätssignal erzeugt, das einen Wechselstrom mit einer Frequenz von etwa 10 kHz bis etwa 30 kHz und einem maximalen Stromfluss von bis zu etwa 10 mA umfasst.

15. Gewebeeindämmungs- und -entnahmesystem nach Anspruch 1, wobei der Gewebestückzerkleinerer (30) ferner ein Gehäuse (206) umfasst, das funktional mit dem Gewebeschneider (34) verbunden ist, und eine Kanüle (38), die an dem Gehäuse (206) befestigt ist, eine längliche hohle Ausgestaltung aufweist und ein inneres Lumen (39) umfasst, das sich über dessen Länge erstreckt und über dem Gewebeschneider (34) angeordnet ist.

**Revendications**

1. Système de confinement et d'ablation de tissu (10), comprenant :

un réceptacle à tissu (40) comprenant une couche conductrice (47) qui comprend un élément conducteur (46), un volume intérieur (42) et une ouverture (43),
un réducteur de tissu de la masse (30) comprenant un organe de coupe de tissu (34) doté d'une lame de coupe de tissu (36) conçue pour être conductrice,
un moteur (51) couplé de manière fonctionnelle à la lame de coupe de tissu (36) du réducteur de tissu de la masse (30), et
un système de détection de contact (70) comprenant :

un circuit de détection (75) qui est couplé de manière fonctionnelle à la lame de coupe de tissu (36) et à l'élément conducteur (46) et qui est conçu pour générer un signal de continuité entre la lame de coupe de tissu (36) et l'élément conducteur (46) et mesurer une valeur d'impédance entre la lame de coupe de tissu (36) et l'élément conducteur (46), et

un dispositif de commande (80) qui est couplé de manière fonctionnelle au moteur (51) et qui est conçu pour interrompre l'actionnement du moteur (51) et de la lame de coupe de tissu (36) chaque fois que l'impédance entre la lame de coupe de tissu (36) et l'élément conducteur (46) est inférieure ou égale à une valeur seuil d'impédance prédéterminée.

2. Système de confinement et d'ablation de tissu selon la revendication 1, dans lequel le dispositif de commande (80) est conçu pour émettre un signal d'avertissement chaque fois que l'impédance entre la lame de coupe de tissu (36) et l'élément conducteur (46) est inférieure ou égale à la valeur seuil d'impédance prédéterminée.

3. Système de confinement et d'ablation de tissu selon la revendication 2, comprenant en outre un émetteur de signal sonore (190), le dispositif de commande (80) étant conçu pour émettre un signal d'avertissement sonore à partir de l'émetteur de signal sonore (190) chaque fois que l'impédance entre la lame de coupe de tissu (36) et l'élément conducteur (46) est inférieure ou égale à la valeur seuil d'impédance prédéterminée.

4. Système de confinement et d'ablation de tissu selon la revendication 1, dans lequel le système de détection de contact (70) comprend en outre une borne de lame (135) qui est couplée de manière fonctionnelle au dispositif de commande (80) et à la lame de coupe de tissu (36) au moyen d'un conduite conductrice (74), et une borne de réceptacle (137) qui est couplée de manière fonctionnelle au dispositif de commande (80) et à l'élément conducteur (46) du réceptacle à tissu (40) à l'aide d'une conduite conductrice (72).

5. Système de confinement et d'ablation de tissu selon la revendication 1, dans lequel le dispositif de commande (80) comprend une carte de circuit imprimé de console (82) et un dispositif d'entraînement de moteur (84) couplé de manière fonctionnelle au moteur (51).

6. Système de confinement et d'ablation de tissu selon la revendication 5, dans lequel le dispositif de commande (80) comprend en outre un générateur de signaux (120), un processeur (86) et une mémoire (88) couplée de manière fonctionnelle au processeur (86).

7. Système de confinement et d'ablation de tissu selon la revendication 1, dans lequel le système de détection de contact (70) comprend en outre une alimentation électrique (112) couplée de manière fonctionnelle au dispositif de commande (80).

8. Système de confinement et d'ablation de tissu selon la revendication 1, dans lequel le réceptacle à tissu (40) comprend en outre une deuxième couche non conductrice (102) disposée sur une surface intérieure (196) de la couche conductrice (47).

9. Système de confinement et d'ablation de tissu selon la revendication 8, dans lequel le réceptacle à tissu (40) comprend en outre une troisième couche non conductrice (198) disposée sur une surface extérieure (200) de la couche conductrice (47).

10. Système de confinement et d'ablation de tissu selon la revendication 1, dans lequel la couche conductrice (47) comprend un tissage composite (234) comprenant des brins non conducteurs (232) entrelacés avec des brins conducteurs (230) qui comprennent l'élément conducteur (46).

11. Système de confinement et d'ablation de tissu selon la revendication 1, dans lequel la couche conductrice (47) comprend une fine couche flexible de matériau polymère non conducteur et un motif d'encre conductrice (95) imprimé sur une surface extérieure (200) de celle-ci.

12. Système de confinement et d'ablation de tissu selon la revendication 1, dans lequel la couche conductrice (47) et son élément conducteur (46) comprennent un treillis métallique (100).

13. Système de confinement et d'ablation de tissu selon la revendication 1, dans lequel l'organe de coupe de tissu (34) comprend un tube allongé (202) doté d'une lumière intérieure (37) s'étendant sur toute sa longueur et la lame de coupe de tissu (36) comprend une extrémité distale affûtée du tube allongé (202).

14. Système de confinement et d'ablation de tissu selon la revendication 1, dans lequel le circuit de détection (75) est conçu pour générer un signal de continuité comprenant un courant alternatif ayant une fréquence d'environ 10 kHz à environ 30 kHz et une intensité maximale pouvant atteindre environ 10 mA.

15. Système de confinement et d'ablation de tissu selon la revendication 1, dans lequel le réducteur de tissu de la masse (30) comprend en outre un boîtier (206) couplé de manière fonctionnelle à l'organe de coupe de tissu (34) et une canule (38) qui est fixée au boîtier (206), qui présente une configuration creuse allongée avec une lumière intérieure (39) qui s'étend sur sa longueur, et qui est disposée par-dessus l'organe de coupe de tissu (34).

FIG. 1

FIG. 2

FIG. 3

FIG. 4

FIG. 5

EP 4 473 924 B1

FIG. 6

FIG. 7

FIG. 8

FIG. 9

FIG. 10

FIG. 11

FIG. 12

FIG. 13

EP 4 473 924 B1

35

FIG. 15

FIG. 14

EP 4 473 924 B1

FIG. 16

FIG. 17

FIG. 18

FIG. 19

FIG. 20

FIG. 21

FIG. 22

FIG. 23

FIG. 24

FIG. 25

FIG. 26

254

34

255

FIG. 27

FIG. 28

FIG. 29

FIG. 30

EP 4 473 924 B1

FIG. 31

FIG. 32

FIG. 33

FIG. 34

FIG. 35

**EP 4 473 924 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62886473, Joseph N. Jones
- US 63006360, Joseph N. Jones
- WO 2018047180 A1
- WO 2016144170 A1
- WO 2019083896 A1
- US 169884
- US 758358